# EUROPEAN PATENT APPLICATION

(11) **EP 4 487 780 A1**
(43) Date of publication of application: **08.01.2025**
(21) Application number: 22929986.2
(22) Date of filing: 28.12.2022
(51) Int. Cl.: A61B 6/00, A61B 6/08

(54) **IMAGING ASSISTANCE DEVICE, METHOD FOR OPERATING IMAGING ASSISTANCE DEVICE, AND PROGRAM FOR OPERATING IMAGING ASSISTANCE DEVICE**

(30) Priority: 03.03.2022 JP 2022032926
(71) Applicant: FUJIFILM Corporation, Tokyo 106-8620 (JP)
(72) Inventor: SUGAHARA, Masataka, Tokyo 106-8620 (JP); KOBAYASHI, Takeyasu, Tokyo 106-8620 (JP)
(74) Representative: Dehns Germany Partnerschaft mbB
(86) International application number: PCT/JP2022/048671
(87) International publication number: WO 2023/166844

(57) **Abstract**

An imaging support apparatus includes a processor, and the processor acquires a two-dimensional image obtained by imaging a subject who undergoes radiography via a camera, extracts a portion of interest of the subject included in the two-dimensional image, demarcates an imaging region to be imaged by the radiography in the two-dimensional image based on the portion of interest, determines whether or not an imaging preparation state before the radiography is appropriate based on the imaging region, and outputs information in accordance with a determination result.

## Description

### BACKGROUND OF THE INVENTION

### 1. Field of the Invention

The technology of the present disclosure relates to an imaging support apparatus, an operation method of an imaging support apparatus, and an operation program of an imaging support apparatus.

### 2. Description of the Related Art

JP6548713B discloses a technology of acquiring a two-dimensional image obtained by imaging a subject who undergoes radiography via a camera, and three-dimensional information (distance information and coordinate information of each joint) obtained by detecting the subject who undergoes the radiography via a three-dimensional information detection unit, extracting a portion of interest of the subject from the two-dimensional image and the three-dimensional information, determining whether or not positioning of the subject is appropriate based on the portion of interest, and outputting information in accordance with a determination result.

JP6548713B describes an orbit, an external auditory canal, a position of a neck portion or both shoulders, a shoulder skeletal point, an abdominal skeletal point, a chest center point, the respective joints, and the like as the portions of interest. In addition, as an example of an aspect in which it is determined whether or not the positioning of the subject is appropriate, an aspect is described in which it is determined whether or not an angle of an orbitomeatal line with respect to a gantry is appropriate, an aspect is described in which it is determined whether or not a center of the external auditory canal is deviated from a rotation center of the gantry, and an aspect is described in which it is determined whether or not a degree of inclination of a posture of the subject with respect to an examination table is large.

### SUMMARY OF THE INVENTION

In the technology disclosed in JP6548713B, as described above, both the two-dimensional image and the three-dimensional information are acquired, and the portion of interest is extracted from the two-dimensional image and the three-dimensional information. Therefore, it takes the cost and time and effort to extract the portion of interest.

In addition, in the technology disclosed in JP6548713B, it is determined whether or not the positioning of the subject is appropriate, but in an imaging preparation state before the radiography, it is necessary to determine the appropriateness including other elements such as whether or not an unnecessary part is included in an imaging region to be imaged in the radiography, in addition to the positioning of the subject. In order to determine whether or not the imaging preparation state including various elements is appropriate, the determination based on the portion of interest described in JP6548713B is insufficient.

One embodiment according to the technology of the present disclosure provides an imaging support apparatus, an operation method of an imaging support apparatus, and an operation program of an imaging support apparatus, which can determine whether or not an imaging preparation state is appropriate in a simpler and more sufficient manner.

The present disclosure relates to an imaging support apparatus comprising: a processor, in which the processor acquires a two-dimensional image obtained by imaging a subject who undergoes radiography via a camera, extracts a portion of interest of the subject included in the two-dimensional image, demarcates an imaging region to be imaged by the radiography in the two-dimensional image based on the portion of interest, determines whether or not an imaging preparation state before the radiography is appropriate based on the imaging region, and outputs information in accordance with a determination result.

It is preferable that the processor determines that the imaging preparation state is not appropriate in a case in which an unnecessary part for the radiography is included in the imaging region or a preset peripheral region of the imaging region.

It is preferable that the processor determines that the imaging preparation state is not appropriate in a case in which a deviation between a center of the imaging region and an irradiation center of radiation is equal to or greater than a preset threshold value.

It is preferable that the processor performs control of displaying, on a display, a deviation amount between a center of the imaging region and an irradiation center of radiation in a real space.

It is preferable that the processor performs control of displaying, on a display, the two-dimensional image on which a frame indicating the imaging region is superimposed.

It is preferable that the processor detects an angle of an imaging part to be imaged by the radiography based on the portion of interest, and determines that the imaging preparation state is not appropriate in a case in which the angle is out of a preset range.

It is preferable that the processor detects an angle of an imaging part to be imaged by the radiography based on the portion of interest, and performs control of adjusting an angle of a radiation source that emits radiation with respect to the subject according to the angle.

It is preferable that the processor detects a degree of inclination of a posture of the subject based on the portion of interest, and determines that the imaging preparation state is not appropriate in a case in which the degree of inclination is out of a preset range.

It is preferable that the processor performs control of adjusting positions of a radiation source that emits radiation and a radiation image detector that receives the radiation to detect a radiation image, to reduce a deviation between a center of the imaging region and an irradiation center of the radiation.

It is preferable that the processor performs control of displaying, on a display, adjustment amounts of positions of a radiation source that emits radiation and a radiation image detector that receives the radiation to detect a radiation image, the adjustment amounts being for reducing a deviation between a center of the imaging region and an irradiation center of the radiation.

It is preferable that the processor performs processing of detecting movement of the subject only on the imaging region.

It is preferable that the processor outputs, in a case in which a movement amount of the subject is equal to or greater than a preset threshold value, a fact that the movement amount of the subject is equal to or greater than the threshold value.

It is preferable that the processor performs, in a case in which a movement amount of the subject is equal to or greater than a preset threshold value, control of preventing a radiation source from emitting radiation.

It is preferable that the processor performs control of displaying, on a display, a temporal change in a component on a specific axis of a movement amount of the subject.

It is preferable that the processing of detecting the movement of the subject is processing of calculating a movement amount of an object shown in two pieces of the two-dimensional images captured at different imaging time points.

The present disclosure relates to an operation method of an imaging support apparatus, the method comprising: acquiring a two-dimensional image obtained by imaging a subject who undergoes radiography via a camera; extracting a portion of interest of the subject included in the two-dimensional image; demarcating an imaging region to be imaged by the radiography in the two-dimensional image based on the portion of interest; determining whether or not an imaging preparation state before the radiography is appropriate based on the imaging region; and outputting information in accordance with a determination result.

The present disclosure relates to an operation program of an imaging support apparatus, the program causing a computer to execute a process comprising: acquiring a two-dimensional image obtained by imaging a subject who undergoes radiography via a camera; extracting a portion of interest of the subject included in the two-dimensional image; demarcating an imaging region to be imaged by the radiography in the two-dimensional image based on the portion of interest; determining whether or not an imaging preparation state before the radiography is appropriate based on the imaging region; and outputting information in accordance with a determination result.

According to the technology of the present disclosure, it is possible to provide the imaging support apparatus, the operation method of the imaging support apparatus, and the operation program of the imaging support apparatus, which can determine whether or not the imaging preparation state is appropriate in a simpler and more sufficient manner.

### BRIEF DESCRIPTION OF THE DRAWINGS

Fig. 1 is a diagram showing a radiography system.
Fig. 2 is a diagram showing a radiography room and a control room.
Fig. 3 is a block diagram showing a configuration of a console.
Fig. 4 is a block diagram showing a processing unit of a CPU of the console.
Fig. 5 is a diagram showing processing of an extraction unit.
Fig. 6 is a diagram showing processing of the extraction unit.
Fig. 7 is a diagram showing processing of a demarcation unit.
Figs. 8A and 8B are diagrams showing processing of a determination unit, in which Fig. 8A shows a case in which a hand is not included in an imaging region, and Fig. 8B shows a case in which the hand is included in the imaging region.
Figs. 9A and 9B are diagrams showing processing of the determination unit, in which Fig. 9A shows a case in which a deviation amount between a center of the imaging region and an irradiation center of radiation is less than a threshold value, and Fig. 9B shows a case in which the deviation amount between the center of the imaging region and the irradiation center of the radiation is equal to or greater than the threshold value.
Figs. 10A and 10B are diagrams showing processing of the determination unit, in which Fig. 10A shows a case in which angles formed by a line connecting right and left shoulder joint points and a line connecting right and left hip joint points, and an X-axis are less than a threshold value, and Fig. 10B shows a case in which the angle formed by the line connecting the right and left shoulder joint points or the line connecting the right and left hip joint points, and the X-axis is equal to or greater than the threshold value.
Fig. 11 is a diagram showing an information display screen in a case in which an imaging preparation state is appropriate.
Fig. 12 is a diagram showing an information display screen in a case in which the hand is included in the imaging region.
Fig. 13 is a diagram showing an information display screen in a case in which the deviation amount between the center of the imaging region and the irradiation center of the radiation is equal to or greater than the threshold value.
Fig. 14 is a diagram showing an information display screen in a case in which an angle formed by the line connecting the right and left shoulder joint points or the line connecting the right and left hip joint points, and the X-axis is equal to or greater than the threshold value.
Fig. 15 is a flowchart showing a processing procedure of the console.
Fig. 16 is a diagram showing processing of the determination unit in a case in which the hand is included in a peripheral region of the imaging region.
Fig. 17 is a view showing a state of knee decubitus side surface imaging.
Fig. 18 is a diagram showing processing of the extraction unit in a case of knee decubitus side surface imaging.
Fig. 19 is a diagram showing processing of the demarcation unit in a case of the knee decubitus side surface imaging.
Figs. 20A and 20B are diagrams showing processing of the determination unit in a case of the knee decubitus side surface imaging, in which Fig. 20A shows a case in which the other foot is not included in the imaging region, and Fig. 20B shows a case in which the other foot is included in the imaging region.
Figs. 21A and 21B are diagrams showing processing of the determination unit in a case of the knee decubitus side surface imaging, in which Fig. 21A shows a case in which the deviation amount between the center of the imaging region and the irradiation center of the radiation is less than the threshold value, and Fig. 21B shows a case in which the deviation amount between the center of the imaging region and the irradiation center of the radiation is equal to or greater than the threshold value.
Figs. 22A and 22B are diagrams showing processing of the determination unit in a case of the knee decubitus side surface imaging, in which Fig. 22A shows a case in which an angle formed by the line connecting the hip joint point and the knee j oint point and a line connecting the knee joint point and an ankle joint point is within a set range, and Fig. 22B shows a case in which the angle formed by the line connecting the hip joint point and the knee joint point and the line connecting the knee joint point and the ankle joint point is out of the set range.
Fig. 23 is a diagram showing an information display screen in a case in which the angle formed by the line connecting the hip j oint point and the knee j oint point and the line connecting the knee j oint point and the ankle j oint point is out of the set range.
Fig. 24 is a diagram showing a state of a knee upright front surface imaging using a Rosenberg method.
Fig. 25 is a diagram showing processing of the extraction unit in a case of the knee upright front surface imaging using the Rosenberg method.
Figs. 26A and 26B are diagram showing processing of the determination unit in a case of the knee upright front surface imaging using the Rosenberg method, in which Fig. 26A shows a case in which the angle formed by the line connecting the hip joint point and the knee joint point and the line connecting the knee joint point and the ankle joint point is within the set range, and Fig. 26B shows a case in which the angle formed by the line connecting the hip joint point and the knee joint point and the line connecting the knee joint point and the ankle joint point is out of the set range.
Fig. 27 is a diagram showing a state of chest portion upright side surface imaging.
Fig. 28 is a diagram showing processing of the extraction unit in a case of the chest portion upright side surface imaging.

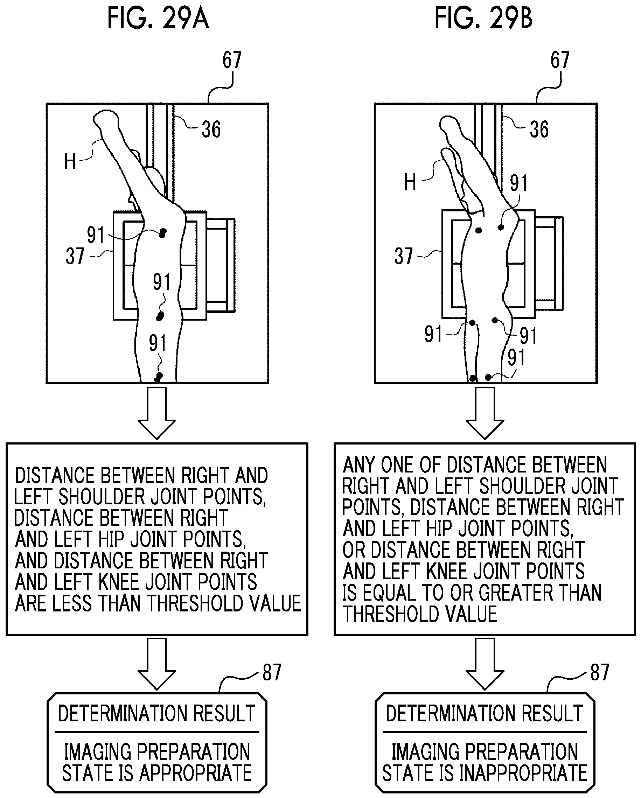

Figs. 29A and 29B are diagrams showing processing of the determination unit in a case of the chest portion upright side surface imaging, in which Fig. 29A shows a case in which a distance between the right and left shoulder joint points, a distance between the right and left hip joint points, and a distance between the right and left knee joint points are within a set range, and Fig. 29B shows a case in which any one of the distance between the right and left shoulder joint points, the distance between the right and left hip joint points, and the distance between the right and left knee joint points is out of the set range.
Fig. 30 is a diagram showing an information display screen in a case in which any one of the distance between the right and left shoulder joint points, the distance between the right and left hip joint points, or the distance between the right and left knee joint points is out of the set range.
Fig. 31 is a diagram showing a state of head portion decubitus side surface imaging using a Towne method.
Fig. 32 is a diagram showing processing of the extraction unit in a case of the head portion decubitus side surface imaging using the Towne method.
Figs. 33A and 33B are diagrams showing processing of the determination unit in a case of the head portion decubitus side surface imaging using the Towne method, in which Fig. 33A shows a case in which an angle formed by an orbitomeatal line and the X-axis is within a set range, and Fig. 33B shows a case in which the angle formed by the orbitomeatal line and the X-axis is out of the set range.
Fig. 34 is a diagram showing another example of an upright imaging table.
Fig. 35 is a diagram showing an aspect in which a portion of interest is extracted based on a two-dimensional image obtained by imaging a subject who undergoes the chest portion upright front surface imaging from above.
Figs. 36A and 36B are diagrams showing processing of the determination unit in the aspect shown in Fig. 35, in which Fig. 36A shows a case in which right and left elbow joint points are located on the upright imaging table side with respect to the right and left shoulder joint points, and Fig. 36B shows a case in which the right and left shoulder joint points are located on the upright imaging table side with respect to the right and left elbow joint points.
Fig. 37 is a diagram showing an aspect in which the deviation between the center of the imaging region and the irradiation center of the radiation is reduced by performing control of adjusting height positions of a radiation source and an electronic cassette in the chest portion upright front surface imaging.
Fig. 38 is a diagram showing an aspect in which the deviation between the center of the imaging region and the irradiation center of the radiation is reduced by performing control of adjusting horizontal positions of the radiation source and the electronic cassette in the knee decubitus side surface imaging.
Fig. 39 is a diagram showing an aspect in which control of adjusting an angle of the radiation source with respect to the subject according to a bending angle of a knee is performed in the knee upright front surface imaging using the Rosenberg method.
Fig. 40 is a diagram showing a schematic configuration of Embodiment 3_1 in which a movement amount of the subject is detected.
Fig. 41 is a diagram showing a method of detecting the movement amount of the subj ect.
Fig. 42 is a diagram showing a method of detecting the movement amount of the subj ect.
Fig. 43 is a diagram showing an information display screen in a case in which the movement amount of the subject is equal to or greater than a preset threshold value.
Fig. 44 is a diagram showing an aspect in which control of displaying a temporal change in a Y-axis component of the movement amount of the subject on a display is performed.
Fig. 45 is a diagram showing an information display screen on which the temporal change in the Y-axis component of the movement amount of the subject is displayed.
Fig. 46 is a diagram showing an information display screen in a case in which the movement amount of the subject is equal to or greater than the preset threshold value.
Fig. 47 is a diagram showing an aspect in which control of displaying a temporal change in an X-axis component of the movement amount of the subject on the display is performed.
Fig. 48 is a diagram showing an information display screen on which the temporal change in the X-axis component of the movement amount of the subject is displayed.

### DESCRIPTION OF THE PREFERRED EMBODIMENTS

### [Embodiment 1]

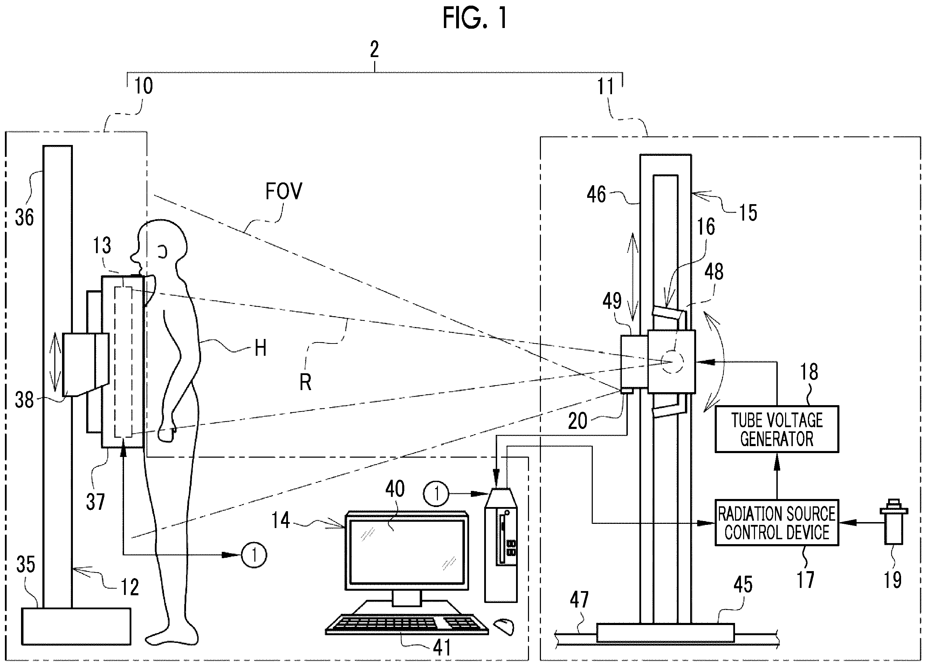

As shown in Fig. 1 as an example, a radiography system 2 is a system that performs radiography of a subject H using radiation R, such as X-rays and γ-rays, and is composed of a radiography apparatus 10 and a radiation generation device 11. The radiography apparatus 10 includes an upright imaging table 12, an electronic cassette 13, and a console 14. The radiation generation device 11 includes a radiation source table 15, a radiation source 16, a radiation source control device 17, a tube voltage generator 18, and an irradiation switch 19. A camera 20 is attached to the radiation source 16.

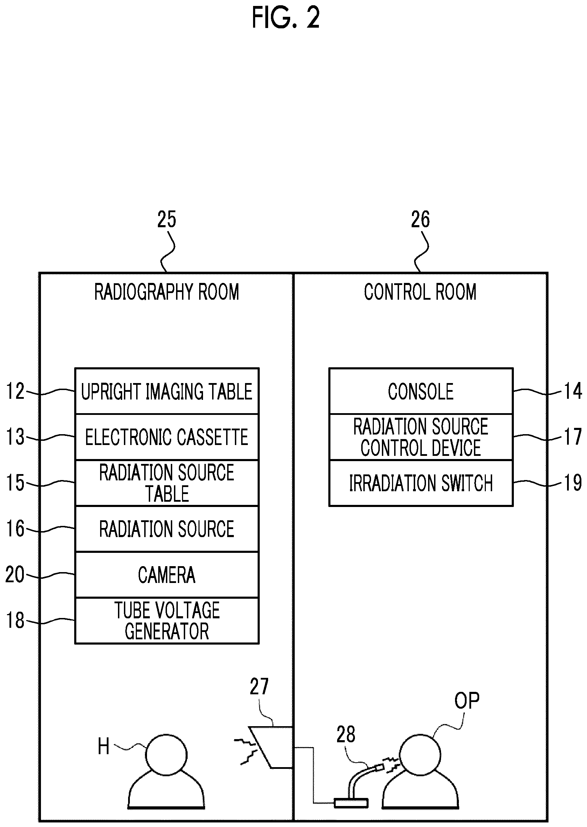

As shown in Fig. 2 as an example, the upright imaging table 12, the electronic cassette 13, the radiation source table 15, the radiation source 16, the camera 20, and the tube voltage generator 18 are installed in a radiography room 25. On the other hand, the console 14, the radiation source control device 17, and the irradiation switch 19 are installed in a control room 26 adjacent to the radiography room 25. A speaker 27 is installed in the radiography room 25, and a microphone 28 is installed in the control room 26. The speaker 27 outputs an utterance sound of an operator OP, such as a medical radiologist, in the control room 26, which is collected by the microphone 28. With the speaker 27 and the microphone 28, it is possible to achieve communication between the subject H in the radiography room 25 and the operator OP in the control room 26. It should be noted that the speaker is also installed in a waiting room for the subject H. Through the speaker and the microphone 28, the operator OP makes an announcement for guiding the subject H, whose an order of the radiography has come, from the waiting room to the radiography room 25.

Returning to Fig. 1, the upright imaging table 12 is an imaging table for performing the radiography of the subject H in an upright posture. The upright imaging table 12 includes a seat 35 installed on a floor surface of the radiography room 25, a support column 36 extending in a height direction from the seat 35, and a holder 37 that holds the electronic cassette 13 inside. The holder 37 is connected to the support column 36 via a connecting part 38. The connecting part 38 and the holder 37 are moved up and down with respect to the support column 36 by a motor or the like in accordance with an imaging part or a physique of the subj ect H. A position at which the holder 37 is moved up and down with respect to the support column 36 is detected by, for example, a linear encoder. The holder 37 can be moved up and down from the control room 26 through the console 14.

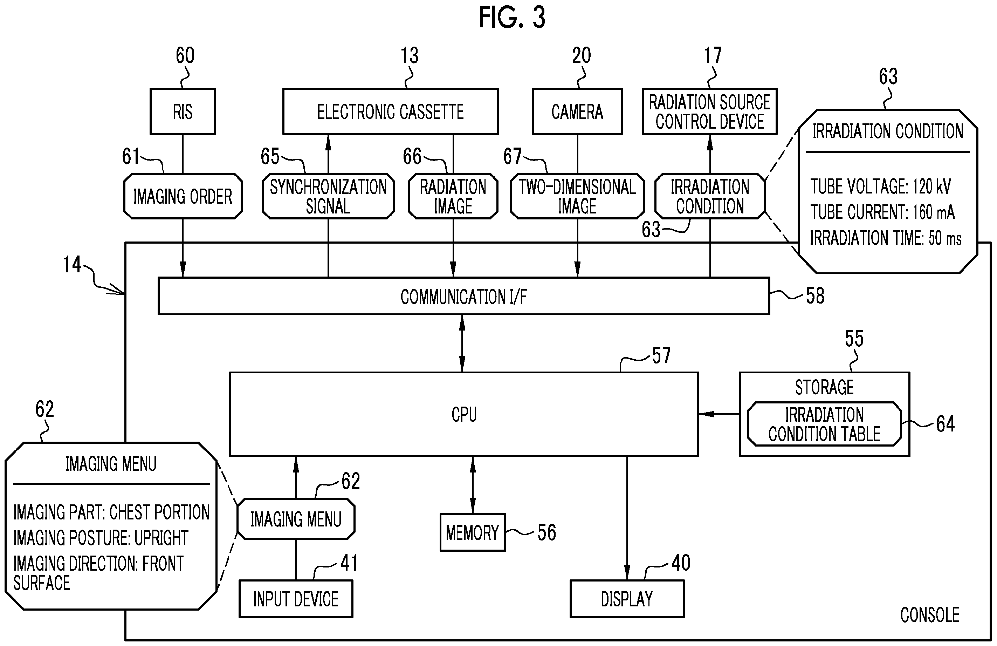

The electronic cassette 13 is a portable radiation image detector that detects a radiation image 66 (see Fig. 3) corresponding to the radiation R transmitted through the subject H. The electronic cassette 13 is connected to the console 14 in a communicable manner by wire or wirelessly. The electronic cassette 13 is accommodated in the holder 37 of the upright imaging table 12 and used. In addition, the electronic cassette 13 can be used by being removed from the holder 37 and held by the subject H, or by being inserted under the subject H lying down on a bed in a hospital room. The electronic cassette 13 is an example of a "radiation image detector" according to the technology of the present disclosure.

The electronic cassette 13 includes a detection panel in which a plurality of pixels accumulating charges corresponding to the radiation R are arranged in a two-dimensional matrix. The detection panel is also called a flat panel detector (FPD). In a case in which the irradiation with the radiation R is started, the detection panel starts an accumulation operation of accumulating the charge in the pixel. In a case in which the irradiation with the radiation R ends, the detection panel starts a readout operation of reading out the charge accumulated in the pixel as an electric signal.

The console 14 is, for example, a desktop personal computer, and is an example of an "imaging support apparatus" according to the technology of the present disclosure. The console 14 includes a display 40 that displays various screens, and an input device 41 that includes a keyboard, a mouse, and the like, and receives an operation instruction of the operator OP. The console 14 transmits various signals to the electronic cassette 13. In addition, the console 14 receives the radiation image 66 from the electronic cassette 13. The console 14 displays the radiation image 66 on the display 40. The display 40 is an example of a "display" according to the technology of the present disclosure. It should be noted that the console 14 may be a laptop personal computer, a tablet terminal, or the like.

The radiation source table 15 includes a seat 45 and a support column 46 that extends in a height direction from the seat 45. The seat 45 is connected to a rail 47 laid on the floor surface of the radiography room 25. The rail 47 is linear and is parallel to a normal line of a detection surface of the radiation R of the electronic cassette 13 accommodated in the holder 37. The seat 45 and the radiation source table 15, can be parallel-moved along the rail 47 by the motor or the like. In this way, the radiation source table 15 moves parallel along the rail 47, so that a source to image receptor distance (SID), which is a distance from a generation point of the radiation R to the detection surface of the radiation R of the electronic cassette 13, is changed. A position of the support column 46 with respect to the rail 47 is detected by the linear encoder, for example. In the same manner as the moving up and down of the holder 37 or the like, the parallel movement of the radiation source 16 can be performed from the control room 26 through the console 14.

The radiation source 16 is attached to the support column 46. The radiation source 16 is rotated about an axis perpendicular to the paper surface with respect to the support column 46 by the motor or the like in order to adjust an incidence angle of the radiation R to the subject H. A rotation angle of the radiation source 16 is detected by, for example, a rotary encoder or a potentiometer. In addition, the radiation source 16 is moved up and down with respect to the support column 46 by the motor or the like in accordance with the imaging part or the physique of the subject H. A position at which the radiation source 16 is moved up and down with respect to the support column 46 is also detected by, for example, the linear encoder. In the same manner as the moving up and down of the holder 37, the rotation and moving up and down of the radiation source 16 can also be performed from the control room 26 through the console 14.

The radiation source 16 includes a radiation tube 48 and an irradiation field limiter 49. The radiation tube 48 is provided with a filament, a target, a grid electrode, and the like (all of which are not shown). A voltage is applied between the filament, which is a cathode, and the target, which is an anode. The voltage applied between the filament and the target is called a tube voltage. The filament releases thermoelectrons corresponding to the applied tube voltage toward the target. The target emits the radiation R by collision of the thermoelectrons from the filament. The grid electrode is disposed between the filament and the target. The grid electrode changes a flow rate of the thermoelectrons from the filament toward the target in accordance with the applied voltage. The flow rate of the thermoelectrons from the filament toward the target is called a tube current.

The irradiation field limiter 49 is also called a collimeter and limits an irradiation field of the radiation R emitted from the radiation tube 48. The irradiation field limiter 49 has a configuration in which, for example, four shielding plates, such as lead, which shield the radiation R are disposed on respective sides of the quadrangle and an emission opening of the quadrangle that transmits the radiation R is formed in a central portion. The irradiation field limiter 49 changes a size of the emission opening by changing a position of each shielding plate, thereby changing the irradiation field of the radiation R.

The tube voltage generator 18 and the irradiation switch 19 are connected to the radiation source control device 17. The radiation source control device 17 controls an operation of the radiation source 16 in response to various instruction signals from the irradiation switch 19. The irradiation switch 19 is operated in a case in which the operator OP instructs the radiation source 16 to start the irradiation with the radiation R.

An irradiation condition 63 (see Fig. 3) of the radiation R is set in the radiation source control device 17. The irradiation condition 63 is the tube voltage, the tube current, and the irradiation time of the radiation R applied to the radiation tube 48 (see Fig. 3). In a case in which the instruction to start the irradiation with the radiation R is given by the operation of the irradiation switch 19, the radiation source control device 17 operates the tube voltage generator 18 in accordance with the set irradiation condition 63 to emit the radiation R from the radiation tube 48. The radiation source control device 17 stops the irradiation with the radiation R from the radiation tube 48 in a case in which the irradiation time set in the irradiation condition 63 elapses after the irradiation with the radiation R is started. The tube voltage generator 18 generates the tube voltage by boosting an input voltage with a transformer. The tube voltage generated by the tube voltage generator 18 is supplied to the radiation tube 48 through a voltage cable (not shown).

It should be noted that the irradiation with the radiation R may end by an auto exposure control (AEC) function. The AEC function is a function of detecting the dose of the radiation R during the irradiation with the radiation R, and stopping the irradiation with the radiation R from the radiation tube 48 at a point in time at which a cumulative dose which is an integrated value of the detected dose, reaches a preset target dose. In this case, the detection panel of the electronic cassette 13 starts the readout operation in a case in which the cumulative dose of the radiation R reaches the target dose.

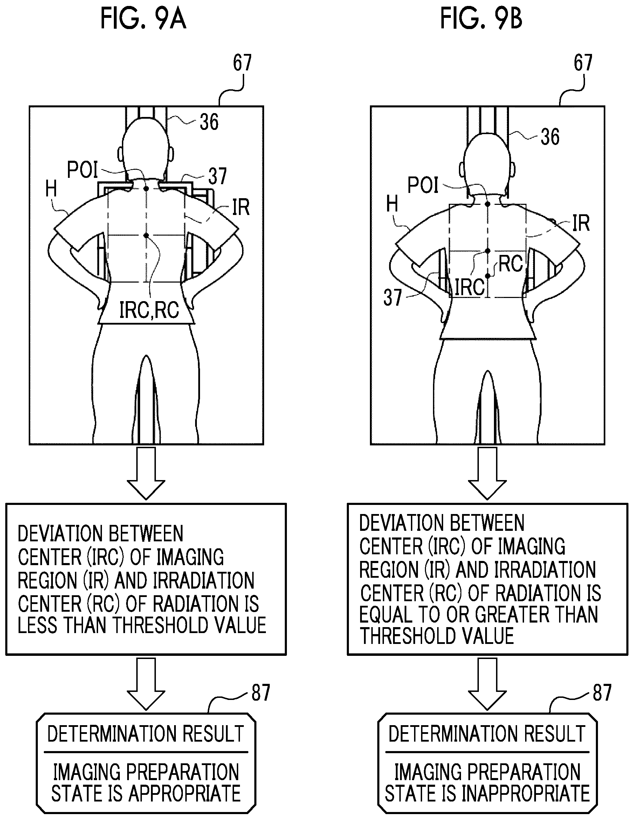

The camera 20 is a digital camera that captures a two-dimensional image 67 (see Fig. 3) that is a digital optical image. The camera 20 is attached to the center of a distal end of the irradiation field limiter 49 of the radiation source 16. The camera 20 is connected to the console 14 in a communicable manner by wire or wirelessly. The camera 20 images the subject H who stands in front of the upright imaging table 12 for the radiography in accordance with an imaging instruction from the console 14. The imaging instruction on the two-dimensional image 67 to the camera 20 via the console 14 is performed by the operator OP, for example, after the subject H is guided from the waiting room to the radiography room 25 and the subject H is made to stand in front of the upright imaging table 12. The camera 20 transmits the captured two-dimensional image 67 to the console 14. The camera 20 is adjusted in its disposition or the like such that a specific point of the two-dimensional image 67, for example, a center of the two-dimensional image 67 (center of a field of view (FOV) of the camera 20) matches an irradiation center RC (see Figs. 9A and 9B and the like) of the radiation R. It should be noted that the camera 20 may be built in the irradiation field limiter 49.

As shown in Fig. 3 as an example, the console 14 comprises a storage 55, a memory 56, a central processing unit (CPU) 57, and a communication interface (I/F) 58, in addition to the display 40 and the input device 41 described above. The display 40, the input device 41, the storage 55, the memory 56, the CPU 57, and the communication I/F 58 are connected to each other via a busline (not shown). The storage 55, the memory 56, the CPU 57, and the busline are examples of a "computer" according to the technology of the present disclosure.

The storage 55 is a hard disk drive built in the computer constituting the console 14 or connected to the computer through a cable or a network. In the storage 55, a control program, such as an operating system, various application programs, various data associated with such programs, and the like are stored. It should be noted that a solid state drive may be used instead of the hard disk drive.

The memory 56 is a work memory for the CPU 57 to execute processing. The CPU 57 loads the program stored in the storage 55 into the memory 56 and executes the processing in accordance with the program. As a result, the CPU 57 controls each unit of the computer in an integrated manner. The CPU 57 is an example of a "processor" according to the technology of the present disclosure. It should be noted that the memory 56 may be built in the CPU 57. The communication I/F 58 controls transmission of various types of information with an external device, such as the electronic cassette 13.

The CPU 57 receives an imaging order 61 from the radiology information system (RIS) 60 via the communication I/F 58. In the imaging order 61, a subject identification data (ID) for identifying the subject H, an instruction on an imaging technique by a doctor or the like of a medical department who has issued the imaging order 61, and the like are registered. The CPU 57 displays the imaging order 61 on the display 40 in response to the operation of the operator OP by the input device 41. The operator OP confirms a content of the imaging order 61 through the display 40.

The CPU 57 displays a plurality of types of imaging menus 62 on the display 40 in a manner in which a plurality of types of imaging menus 62 can be selected. The imaging menu 62 defines an imaging technique in which an imaging part of the subject H, an imaging posture of the subject H, and an imaging direction of the subject H are set as one set, such as "chest portion, upright, front surface". The imaging part includes a head portion, a neck portion, an abdomen portion, a waist portion, a shoulder, an elbow, a hand, a knee, an ankle, and the like, in addition to the chest portion. The imaging posture includes decubitus, sitting, and the like, in addition to the upright. The imaging direction includes a back surface, a side surface, and the like, in addition to the front surface. The operator OP operates the input device 41 to select one imaging menu 62 that matches the imaging technique designated in the imaging order 61 from among the plurality of types of imaging menus 62. As a result, the CPU 57 receives the imaging menu 62. The CPU 57 reads out the irradiation condition 63 corresponding to the received imaging menu 62 from an irradiation condition table 64 stored in the storage 55. The CPU 57 displays the read out irradiation condition 63 on the display 40. In the irradiation condition table 64, the irradiation conditions 63 corresponding to the various imaging menus 62 are registered. As described above, the irradiation condition 63 is the tube voltage and the tube current applied to the radiation tube 48, and the irradiation time of the radiation R. Instead of the tube current and the irradiation time, a tube current irradiation time product may be set as the irradiation condition 63.

The CPU 57 transmits the set irradiation condition 63 to the radiation source control device 17 via the communication I/F 58. In addition, although not shown, in a case in which the radiation source control device 17 is instructed to start the irradiation with the radiation R through the irradiation switch 19, the CPU 57 receives an irradiation start signal indicating that the irradiation with the radiation R is started from the radiation source control device 17. In a case in which the irradiation start signal is received, the CPU 57 transmits a synchronization signal 65 indicating that the irradiation with the radiation R is started to the electronic cassette 13. Further, the CPU 57 receives an irradiation end signal indicating that the irradiation with the radiation R ends from the radiation source control device 17. In a case in which the irradiation end signal is received, the CPU 57 transmits the synchronization signal 65 indicating that the irradiation with the radiation R ends to the electronic cassette 13.

In a case in which the synchronization signal 65 indicating that the irradiation with the radiation R is started is received from the console 14, the electronic cassette 13 causes the detection panel to start the accumulation operation. In addition, in a case in which the synchronization signal 65 indicating that the irradiation with the radiation R ends is received from the console 14, the electronic cassette 13 causes the detection panel to start the readout operation. It should be noted that the electronic cassette 13 may be provided with a function of detecting the start of the irradiation with the radiation R and the end of the irradiation with the radiation R, the detection panel may be caused to start the accumulation operation in a case in which the start of the irradiation with the radiation R is detected by this function, and the detection panel may be caused to start the readout operation in a case in which the end of the irradiation with the radiation R is detected.

The CPU 57 receives the radiation image 66 from the electronic cassette 13 via the communication I/F 58. The CPU 57 performs various types of image processing on the radiation image 66 and then displays the radiation image 66 on the display 40 and provides the radiation image 66 for viewing by the operator.

In addition, although not shown, the CPU 57 transmits the imaging instruction to the camera 20 via the communication I/F 58. The CPU 57 receives the two-dimensional image 67 captured by the camera 20 in accordance with the imaging instruction.

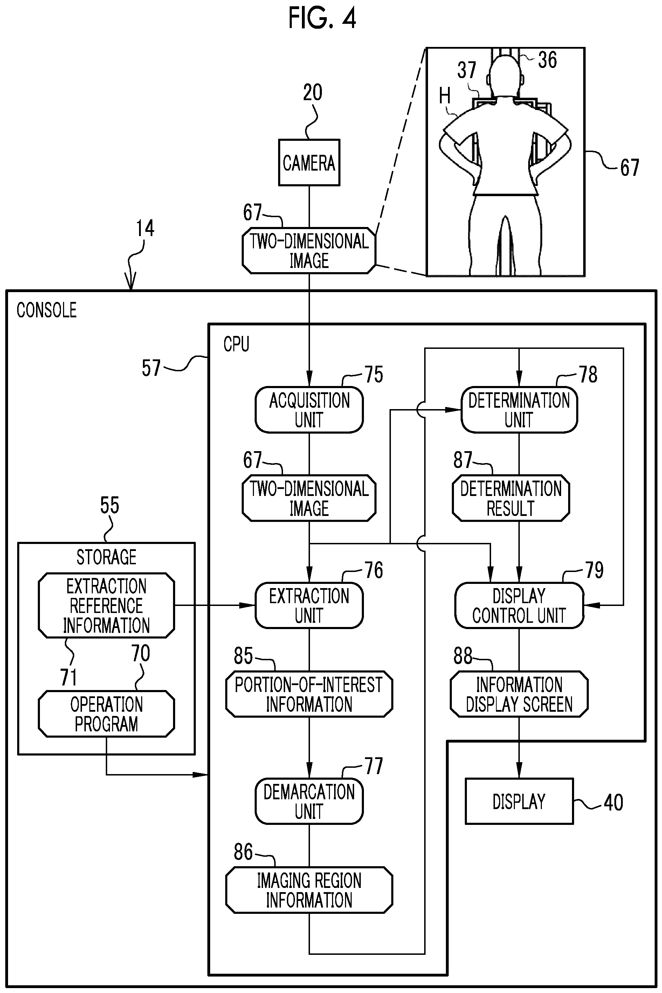

As an example, as shown in Fig. 4, an operation program 70 is stored in the storage 55. The operation program 70 is an application program causing the computer to function as the imaging support apparatus. That is, the operation program 70 is an example of an "operation program of an imaging support apparatus" according to the technology of the present disclosure. The storage 55 also stores extraction reference information 71 and the like.

In a case in which the operation program 70 is activated, the CPU 57 functions as an acquisition unit 75, an extraction unit 76, a demarcation unit 77, a determination unit 78, and a display control unit 79 in cooperation with the memory 56 and the like.

The acquisition unit 75 sequentially acquires the two-dimensional images 67 output from the camera 20 at a predetermined frame rate. The two-dimensional image 67 shows the back surface of the subject H in front of the upright imaging table 12 for the radiography from the top of the head portion to the lower portion of the knee (the entire upper body and a part of the lower body). The acquisition unit 75 outputs the two-dimensional image 67 to the extraction unit 76, the determination unit 78, and the display control unit 79.

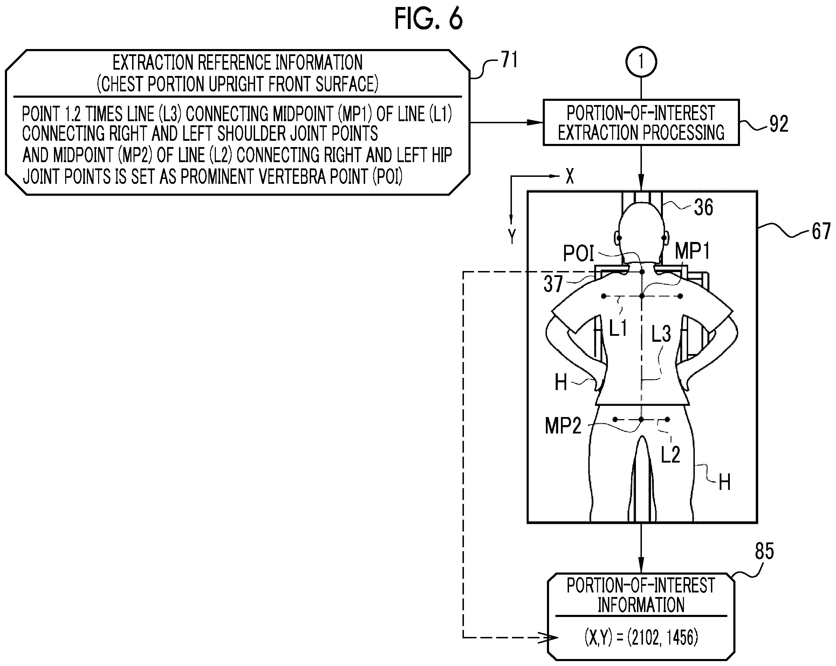

The extraction reference information 71 is prepared for each imaging menu 62. The extraction unit 76 reads out the extraction reference information 71 in accordance with the selected imaging menu 62 from the storage 55. The extraction unit 76 extracts a portion of interest POI (see Fig. 6) of the subject H included in the two-dimensional image 67 with reference to the extraction reference information 71. The portion of interest POI is an actual portion of interest in a human body in a case in which the operator OP positions the subject H. The extraction unit 76 outputs portion-of-interest information 85, which is information on the extracted portion of interest POI, to the demarcation unit 77.

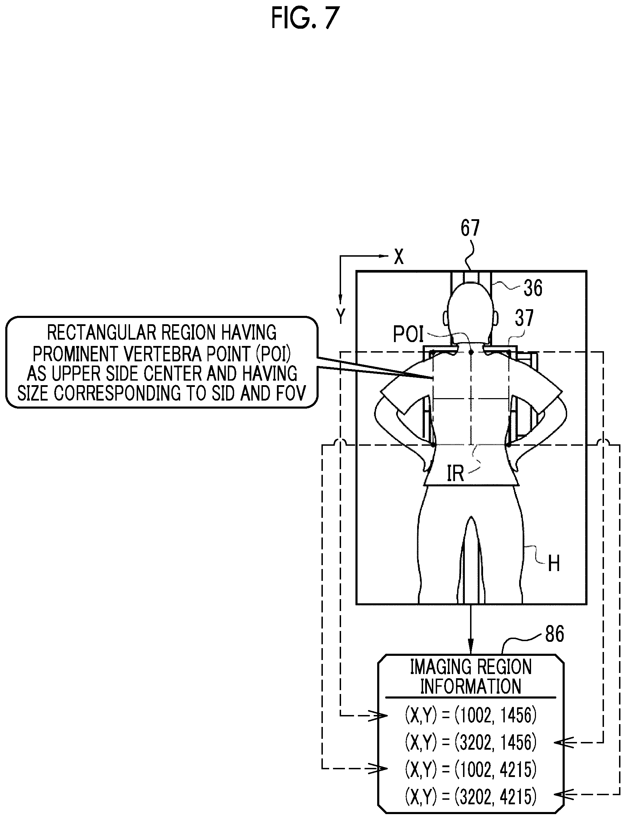

The demarcation unit 77 demarcates an imaging region IR (see Fig. 7) to be imaged by the radiography in the two-dimensional image 67 based on the portion-of-interest information 85. The imaging region IR is a region preset in accordance with the imaging menu 62, and is a region of a human body that should be included in the radiation image 66 in the imaging menu 62. The demarcation unit 77 outputs imaging region information 86, which is information on the demarcated imaging region IR, to the determination unit 78 and the display control unit 79.

The determination unit 78 determines whether or not an imaging preparation state before the radiography is appropriate based on the two-dimensional image 67 and the imaging region information 86. The determination unit 78 outputs a determination result 87 as to whether or not the imaging preparation state is appropriate, to the display control unit 79.

The display control unit 79 generates an information display screen 88. The display control unit 79 performs control of displaying the information display screen 88 on the display 40. It should be noted that, although not shown, in addition to the processing units 75 to 79, the CPU 57 is configured with a reception unit that receives the imaging order 61 from the RIS 60, a reception unit that receives the imaging menu 62 from the input device 41, an image processing unit that acquires the radiation image 66 from the electronic cassette 13 to perform various types of image processing, a setting unit that sets the irradiation condition 63 in the radiation source control device 17, and the like.

Hereinafter, a case of chest portion upright front surface imaging will be described as an example.

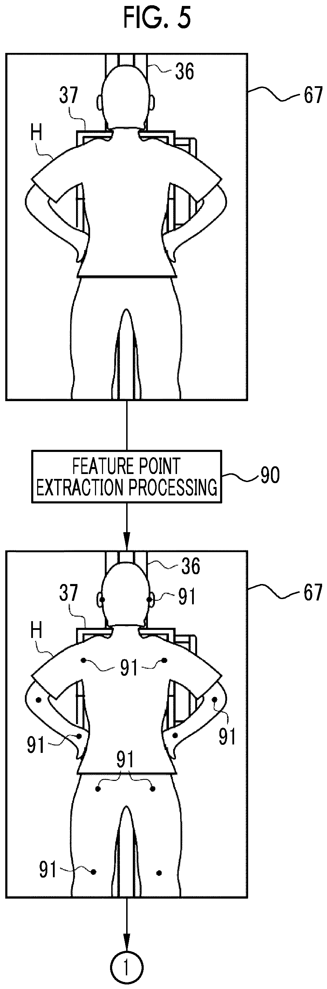

As shown in Fig. 5 as an example, the extraction unit 76 performs feature point extraction processing 90 on the two-dimensional image 67. The feature point extraction processing 90 is processing of extracting a feature point 91 of the subject H shown in the two-dimensional image 67 by using a well-known image recognition technology or a machine learning model. The feature points 91 are right and left external auditory canal points, right and left shoulder joint points, the right and left elbow joint points, right and left wrist joint points, right and left hip joint points, and right and left knee joint points from the top. An external auditory canal is a so-called ear canal, and the external auditory canal point is a center point of the ear canal. The shoulder joint point is a connection point between a shoulder blade and a humerus. The elbow joint point is a connection point between the humerus and a forearm bone. The wrist joint point is a connection point between the forearm bone and a carpal bone. The hip joint point is a connection point between a hip bone and a femoral bone. The knee joint point is a connection point between the femoral bone and a shinbone.

As shown in Fig. 6 as an example, the extraction unit 76 performs portion-of-interest extraction processing 92 on the two-dimensional image 67 following the feature point extraction processing 90. The portion-of-interest extraction processing 92 is processing of extracting a portion of interest POI from the feature point 91 with reference to the extraction reference information 71. The portion of interest POI in the present example is a center point of the prominent vertebra (hereinafter, referred to as a prominent vertebra point). Therefore, the extraction reference information 71 is information for specifying the prominent vertebra. Specifically, the extraction reference information 71 is a content in which a point 1.2 times a length of a line L3, which connects a midpoint MP1 of a line L1 connecting the right and left shoulder joint points and a midpoint MP2 of a line L2 connecting the right and left hip joint points, is set as the prominent vertebra point. Therefore, a length of a line connecting the midpoint MP2 and the prominent vertebra point is 1.2 times the length of the line L3. The extraction unit 76 outputs XY coordinates of the prominent vertebra point extracted in this way to the demarcation unit 77 as the portion-of-interest information 85.

The X-axis of the XY coordinates is an axis parallel to a lateral direction of the two-dimensional image 67, and the Y-axis is an axis parallel to a longitudinal direction of the two-dimensional image 67. Since the lateral direction of the two-dimensional image 67 is parallel to the horizontal direction, the X-axis is also parallel to the horizontal direction. In addition, since the longitudinal direction of the two-dimensional image 67 in a case in which the rotation angle of the radiation source 16 is 0° shown in Fig. 1 is parallel to the vertical direction, the Y-axis is also parallel to the vertical direction in a case in which the rotation angle of the radiation source 16 is 0°.

It should be noted that the numerical value of " 1.2 times" of the extraction reference information 71 is statistically obtained from the data of a large number of unspecified subjects H in the past. For example, the numerical values may be changed in accordance with the attribute of the subject H, such as gender, age, and body type, such as 1.2 times for men, 1.18 times for women, 1.12 times for children, and 1.22 times for height of 180 cm or more.

As shown in Fig. 7 as an example, the demarcation unit 77 demarcates, as the imaging region IR, a rectangular region having the prominent vertebra point as the center and having a size corresponding to the SID and the FOV of the camera 20. The demarcation unit 77 outputs the XY coordinates of the four vertices of the imaging region IR to the determination unit 78 and the like as the imaging region information 86. It should be noted that the XY coordinates of the diagonal point of the imaging region IR may be output as the imaging region information 86.

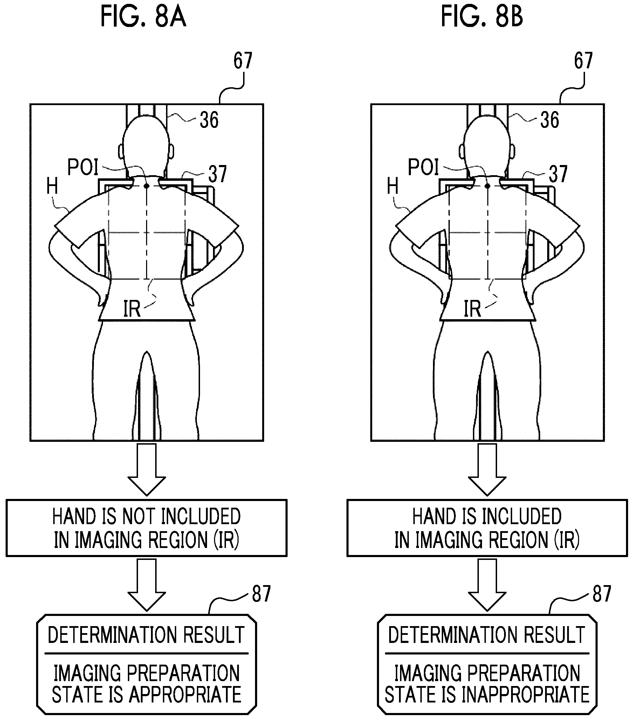

As shown in Figs. 8A and 8B as an example, the determination unit 78 determines whether or not the imaging preparation state is appropriate, depending on whether or not the hand of the subject H is included in the imaging region IR. Whether or not the hand of the subject H is included in the imaging region IR is determined, for example, depending on whether or not a distance between the right and left wrist joint points and the imaging region IR is less than a threshold value. As shown in Fig. 8A, in a case in which the hand is not included in the imaging region IR, the determination unit 78 determines that the imaging preparation state is appropriate, and outputs the determination result 87 indicating that the imaging preparation state is appropriate. On the other hand, as shown in Fig. 8B, in a case in which the hand is included in the imaging region IR, the determination unit 78 determines that the imaging preparation state is not appropriate, and outputs a determination result 87 indicating that the imaging preparation state is not appropriate (is inappropriate). The hand is an example of an "unnecessary part for the radiography" according to the technology of the present disclosure.

As shown in Figs. 9A and 9B as an example, the determination unit 78 determines whether or not the imaging preparation state is appropriate based on a deviation (distance) between a center IRC of the imaging region IR and the irradiation center RC of the radiation R. As shown in Fig. 9A, in a case in which the deviation between the center IRC of the imaging region IR and the irradiation center RC of the radiation R is less than a threshold value, the determination unit 78 determines that the imaging preparation state is appropriate, and outputs the determination result 87 indicating that the imaging preparation state is appropriate. On the other hand, as shown in Fig. 9B, in a case in which the deviation between the center IRC of the imaging region IR and the irradiation center RC of the radiation R is equal to or greater than the threshold value, the determination unit 78 determines that the imaging preparation state is not appropriate, and outputs the determination result 87 indicating that the imaging preparation state is not appropriate. It should be noted that the threshold value in this case is, for example, 5 cm.

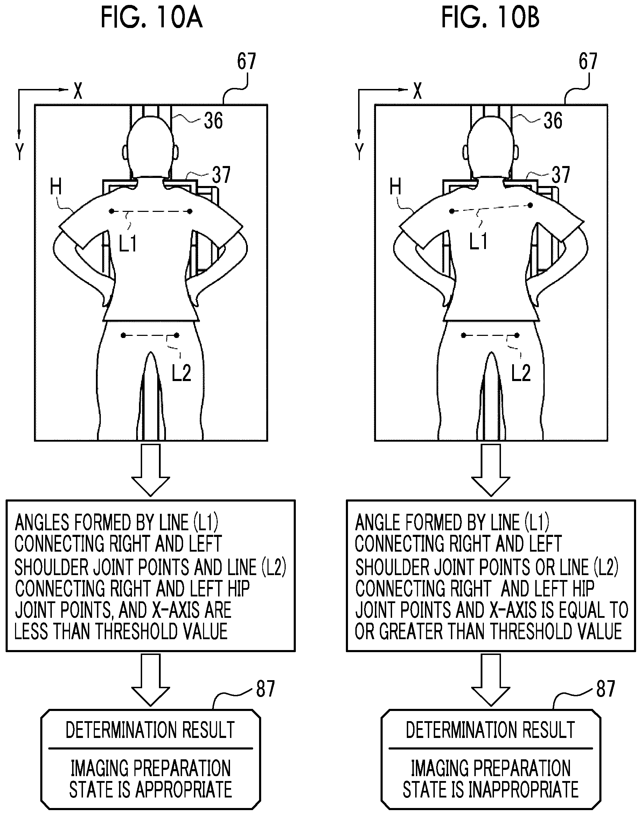

As shown in Figs. 10A and 10B as an example, the determination unit 78 detects a degree of inclination of the posture of the subject H based on the right and left shoulder joint points and the right and left hip joint points. More specifically, the determination unit 78 detects an angles formed by the line L1 connecting the right and left shoulder joint points and the line L2 connecting the right and left hip joint points, and the X-axis, as the degree of inclination of the posture of the subject H. Therefore, the right and left shoulder joint points and the right and left hip joint points are also the feature points 91 and are also examples of a "portion of interest" according to the technology of the present disclosure. In addition, the feature point extraction processing 90 shown in Fig. 5 is also the portion-of-interest extraction processing 92. It should be noted that a difference in the Y coordinate between the right and left shoulder joint points and a difference in the Y coordinate between the right and left hip joint points may be detected as the degree of inclination of the posture of the subject H.

The determination unit 78 determines whether or not the imaging preparation state is appropriate, depending on the degree of inclination of the posture of the subject H. As shown in Fig. 10A, in a case in which the angles formed by the line L1 connecting the right and left shoulder joint points and the X-axis and the line L2 connecting the right and left hip joint points, and the X-axis are both less than the threshold value, the determination unit 78 determines that the imaging preparation state is appropriate, and outputs the determination result 87 indicating that the imaging preparation state is appropriate. On the other hand, as shown in Fig. 10B, in a case in which the angle formed by the line L1 connecting the right and left shoulder joint points or the line L2 connecting the right and left hip joint points, and the X-axis is equal to or greater than the threshold value, the determination unit 78 determines that the imaging preparation state is not appropriate, and outputs the determination result 87 indicating that the imaging preparation state is not appropriate. It should be noted that the threshold value in this case is, for example, ±10°.

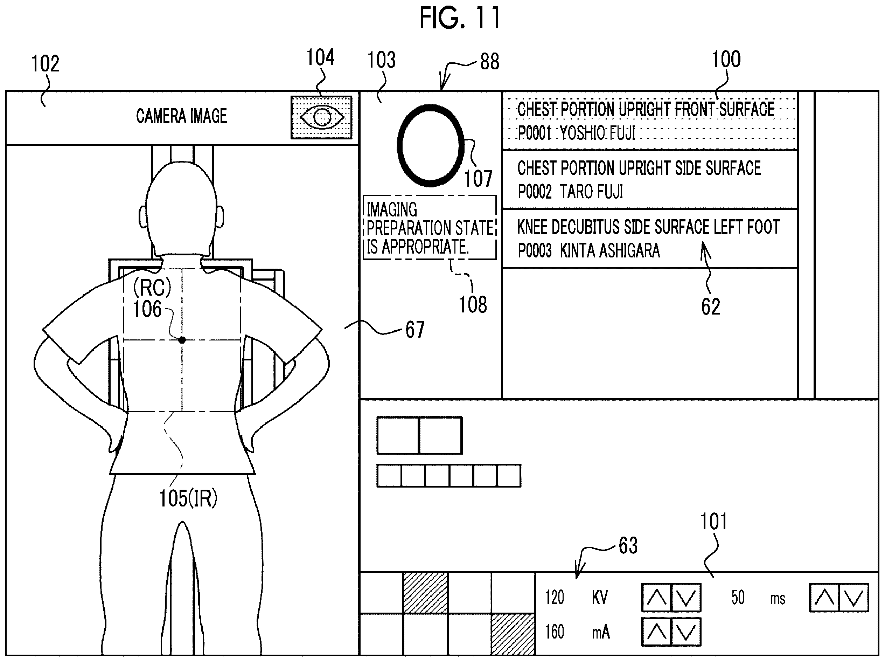

As shown in Figs. 11 to 14 as an example, the information display screen 88 includes a display region 100 of the imaging menu 62 and a display region 101 of the irradiation condition 63. In the display region 100, a set of the imaging menu 62, the subject ID, and the name of the subject H registered so far is displayed side by side. The imaging menu 62 of the currently performed radiography is displayed in a color different from that of the other imaging menus 62, as indicated by hatching. The display region 101 displays the tube voltage, the tube current, and the irradiation time under the irradiation condition 63 in an adjustable state.

The information display screen 88 also has a display region 102 of the two-dimensional image 67 and a display region 103 of the information corresponding to the determination result 87. An imaging instruction button 104 is provided on the upper portion of the display region 102. The imaging instruction button 104 is a turning on/off button. In a case in which the imaging instruction button 104 is in an off state, the imaging instruction on the two-dimensional image 67 is not transmitted to the camera 20. Therefore, the two-dimensional image 67 is not displayed in the display region 102. On the other hand, in a case in which the imaging instruction button 104 is turned on, the imaging instruction on the two-dimensional image 67 is transmitted to the camera 20, and thus the two-dimensional image 67 is displayed in the display region 102. The display control unit 79 displays the two-dimensional image 67 output from the camera 20 at a predetermined frame rate in the display region 102 while sequentially updating the two-dimensional image 67. That is, the two-dimensional image 67 displayed in the display region 102 is a live view image (moving image).

A frame 105 indicating the imaging region IR is displayed in the two-dimensional image 67 of the display region 102 based on the imaging region information 86. A point 106 indicating the irradiation center RC of the radiation R is displayed on the two-dimensional image 67 in the display region 102.

Fig. 11 shows the information display screen 88 in a case in which the determination result 87 indicates that the imaging preparation state is appropriate (in a case of Fig. 8A, Fig. 9A, and Fig. 10A), in all the determinations shown in Figs. 8A to 10B. In this case, a circle mark 107 and a message 108 indicating that the imaging preparation state is appropriate are displayed in the display region 103.

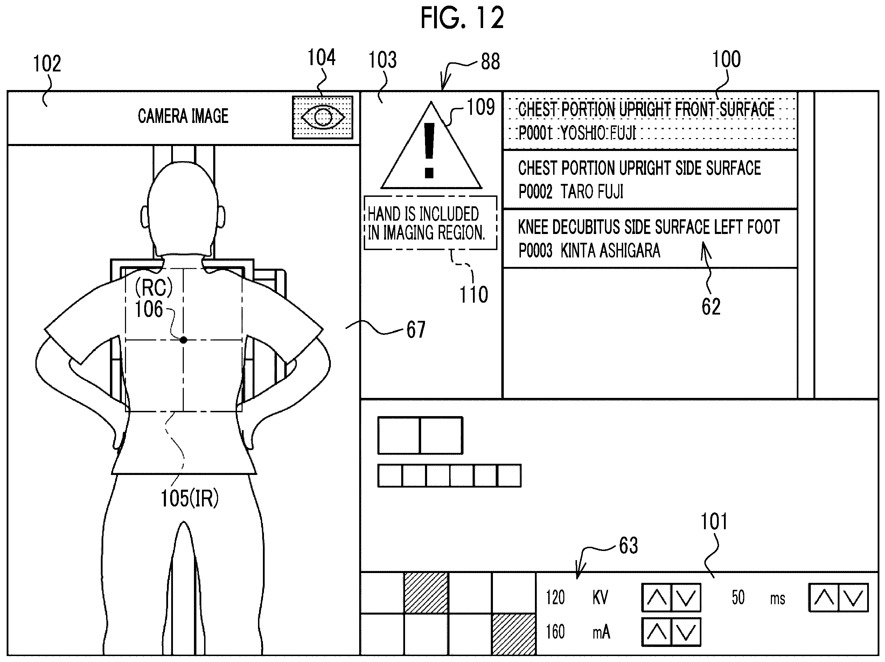

Fig. 12 shows the information display screen 88 in a case in which the determination result 87 indicates that the hand is included in the imaging region IR and that the imaging preparation state is not appropriate (in a case of Fig. 8B), in the determinations shown in Figs. 8A and 8B. In this case, an exclamation mark 109 and a message 110 indicating that the hand is included in the imaging region IR are displayed in the display region 103. The operator OP instructs the subject H to put down the hand by making an announcement to the speaker 27 of the radiography room 25 via the microphone 28.

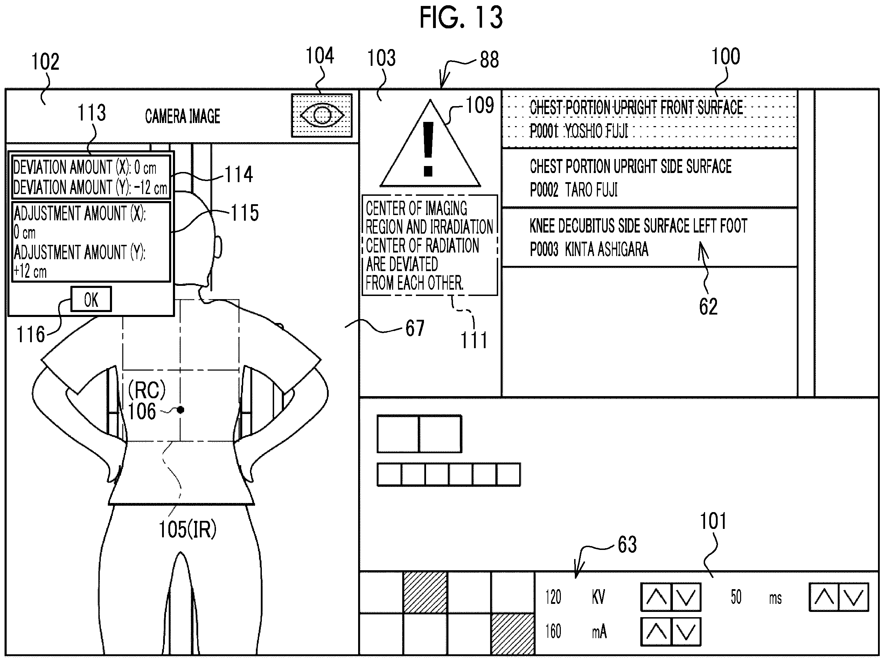

Fig. 13 shows the information display screen 88 in a case in which the determination result 87 indicates that the deviation between the center IRC of the imaging region IR and the irradiation center RC of the radiation R is equal to or greater than the threshold value and that the imaging preparation state is not appropriate (in a case of Fig. 9B), in the determinations shown in Figs. 9A and 9B. In this case, the exclamation mark 109 and a message 111 indicating that the center IRC of the imaging region IR and the irradiation center RC of the radiation R are deviated from each other are displayed in the display region 103.

In this case, as shown in a dialog box 113, the display control unit 79 displays, in the display region 102, deviation amounts 114 between the center IRC of the imaging region IR and the irradiation center RC of the radiation R in the X-axis direction and the Y-axis direction in a real space, which are calculated geometrically from the two-dimensional image 67 and the SID. In addition, as shown in the dialog box 113, the display control unit 79 displays, in the display region 102, adjustment amounts 115 of the electronic cassette 13 and the radiation source 16 in the X-axis direction and the Y-axis direction, the adjustment amounts 115 being for making the deviation between the center IRC of the imaging region IR and the irradiation center RC of the radiation R zero. The display of the dialog box 113 is turned off by selecting an OK button 116. The operator OP operates the console 14 to move the electronic cassette 13 (holder 37) and the radiation source 16 up and down in accordance with the display of the dialog box 113, to reduce the deviation in the height direction between the center IRC of the imaging region IR and the irradiation center RC of the radiation R.

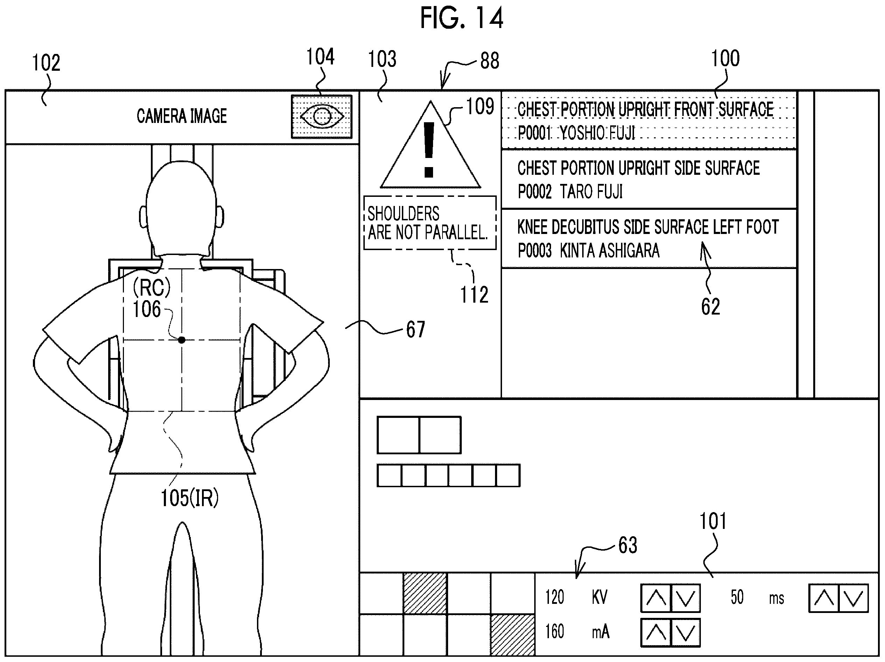

Fig. 14 shows the information display screen 88 in a case in which the determination result 87 indicates that the angle formed by the line L1 connecting the right and left shoulder joint points and the X-axis is equal to or greater than the threshold value and that the imaging preparation state is not appropriate (in a case of Fig. 10B), in the determinations shown in Figs. 10A and 10B. In this case, the exclamation mark 109 and a message 112 indicating that the shoulders are not parallel are displayed in the display region 103. The operator OP instructs the subject H to make the shoulder parallel by making an announcement to the speaker 27 of the radiography room 25 via the microphone 28. Here, the circle mark 107, the messages 108 and 110 to 112, and the exclamation mark 109 are examples of "information in accordance with a determination result" according to the technology of the present disclosure.

It should be noted that, in a case in which a plurality of situations in which the imaging preparation state is not appropriate are caused in combination, a plurality of messages corresponding to the situations caused in combination are displayed in the display region 103. For example, in both the determinations shown in Figs. 8A to 9B, in a case of the determination result 87 indicating that the imaging preparation state is not appropriate (in a case of Figs. 8B and 9B), the message 110 shown in Fig. 12 and the message 111 shown in Fig. 13 are displayed in the display region 103.

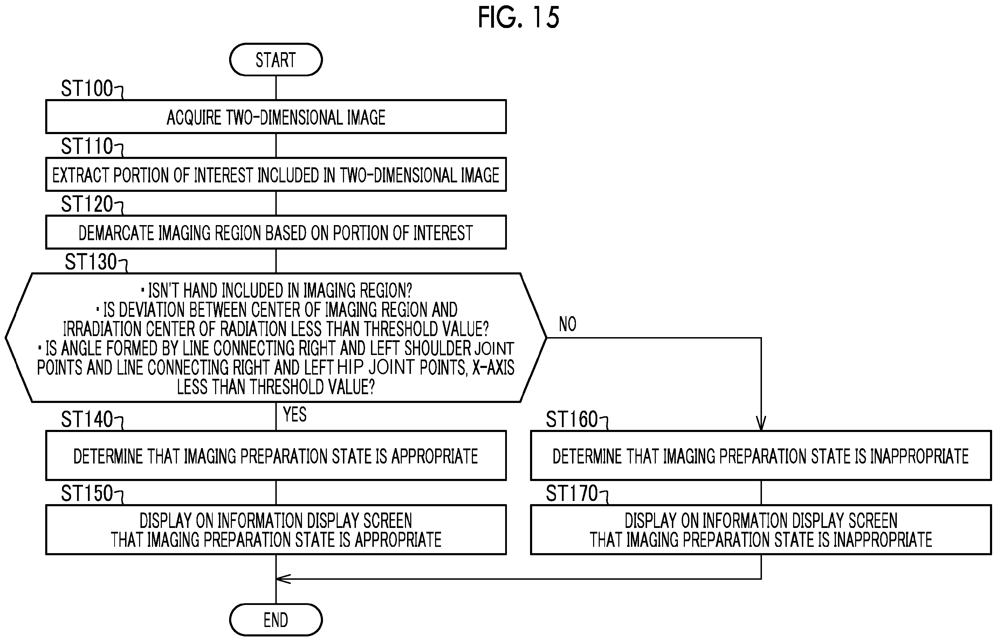

Next, an action with the configuration described above will be described with reference to the flowchart shown in Fig. 15 as an example. Prior to the radiography, the operator OP performs imaging preparation work. The imaging preparation work includes selection of the imaging menu 62, setting of the irradiation condition 63 of the radiation R, positioning of the electronic cassette 13 and the radiation source 16 (adjustment of the height position and the SID), and positioning of the subject H (adjustment of the position and the posture of the subject H with respect to the electronic cassette 13 and the radiation source 16). The operator OP operates the console 14 in the control room 26 to select the imaging menu 62 in accordance with the radiography to be performed, and then sets the irradiation condition 63 for the radiation R. Next, the operator OP transmits the announcement to the speaker of the waiting room through the microphone 28, and guides the subject H from the waiting room to the radiography room 25.

The operator OP instructs the subject H to stand in front of the upright imaging table 12 by making an announcement to the speaker 27 of the radiography room 25 via the microphone 28. The operator OP operates the console 14 to move the holder 37 and the electronic cassette 13 up and down depending on the height of the subject H and to adjust the height position of the electronic cassette 13. In addition, the operator OP operates the console 14 to move the radiation source 16 up and down to move the radiation source 16 to the height position corresponding to the height position of the electronic cassette 13. Further, the operator OP operates the console 14 to move the radiation source table 15 parallel to the rail 47 and to move the radiation source 16 to a position of the SID corresponding to the selected imaging menu 62.

The operator OP makes an announcement to the speaker 27 of the radiography room 25 through the microphone 28. That is, the subject H is prompted to place the jaw on the holder 37, to place the hand on the waist and to protrude the elbow forward, to open the shoulder blades to cover the holder 37 side, and to bring the chest portion into close contact with the holder 37.

The operator OP guides the subject H from the waiting room to the radiography room 25 and to make the subject H stand in front of the upright imaging table 12, and then turns on the imaging instruction button 104 of the information display screen 88 to determine whether or not the imaging preparation state is appropriate by the two-dimensional image 67. As a result, the imaging instruction on the two-dimensional image 67 is transmitted from the console 14 to the camera 20, and the capturing of the two-dimensional image 67 via the camera 20 is started.

In the console 14, the operation program 70 is activated, whereby the CPU 57 functions as the acquisition unit 75, the extraction unit 76, the demarcation unit 77, the determination unit 78, and the display control unit 79. The two-dimensional image 67 from the camera 20 is acquired by the acquisition unit 75 (step ST100). The two-dimensional image 67 is output from the acquisition unit 75 to the extraction unit 76, the determination unit 78, and the display control unit 79.

As shown in Figs. 5 and 6, in the extraction unit 76, the feature point extraction processing 90 and the portion-of-interest extraction processing 92 are performed on the two-dimensional image 67, and the portion of interest POI (here, the prominent vertebra point) is extracted (step ST110). The portion-of-interest information 85 indicating the XY coordinates of the portion of interest POI is output from the extraction unit 76 to the demarcation unit 77.

As shown in Fig. 7, the demarcation unit 77 demarcates the imaging region IR in the two-dimensional image 67 based on the portion of interest POI (step ST120). The imaging region information 86 indicating the XY coordinates of the four vertices of the imaging region IR is output from the demarcation unit 77 to the determination unit 78 and the display control unit 79.

As shown in Figs. 8A to 10B, the determination unit 78 determines whether or not the imaging preparation state is appropriate (step ST130). Specifically, it is determined whether or not the hand is included in the imaging region IR, whether or not the center IRC of the imaging region IR and the irradiation center RC of the radiation R are deviated from each other by the threshold value or greater, and whether or not the angle formed by the line L1 connecting the right and left shoulder joint points or the line L2 connecting the right and left hip joint points, and the X-axis is equal to or greater than the threshold value.

In a case in which the hand is not included in the imaging region IR, the center IRC of the imaging region IR and the irradiation center RC of the radiation R are less than the threshold value, and the angle formed by the line L1 connecting the right and left shoulder joint points and the X-axis of the line L2 connecting the right and left hip joint points is less than the threshold value (YES in step ST130), the determination unit 78 determines that the imaging preparation state is appropriate (step ST140). In this case, as shown in Fig. 11, the circle mark 107 and the message 108 indicating that the imaging preparation state is appropriate are displayed in the display region 103 of the information display screen 88 (step ST150).

On the other hand, in at least any one case of a case in which the hand is included in the imaging region IR, a case in which the center IRC of the imaging region IR and the irradiation center RC of the radiation R are equal to or greater than the threshold value, or a case in which the angle formed by the line L1 connecting the right and left shoulder joint points or the line L2 connecting the right and left hip joint points, and the X-axis is equal to or greater than the threshold value (NO in step ST130), the determination unit 78 determines that the imaging preparation state is not appropriate (is inappropriate) (step ST160). In this case, as shown in Figs. 12 to 14, the exclamation mark 109 and the messages 110 to 112 indicating that the imaging preparation state is not appropriate are displayed in the display region 103 of the information display screen 88 (step ST170). In a case in which the messages 110 to 112 indicating that the imaging preparation state is not appropriate are displayed, the operator OP takes measures to make the imaging preparation state appropriate.

In a case in which the determination unit 78 determines that the imaging preparation state is appropriate, the operator OP instructs the subject H to inhale and hold the breath by making an announcement to the speaker 27 of the radiography room 25 via the microphone 28. Thereafter, the operator OP operates the irradiation switch 19 to instruct the radiation source 16 to start irradiation with the radiation R. As a result, the radiation R is emitted from the radiation source 16 toward the subject H.

The radiation R transmitted through the subject H reaches the electronic cassette 13. Then, the radiation R is detected as the radiation image 66 by the electronic cassette 13. The radiation image 66 is output to the console 14 from the electronic cassette 13. Then, on the console 14, various types of image processing are performed on the radiation image 66 from the electronic cassette 13. Then, the radiation image 66 is displayed in the display region 102 of the information display screen 88 instead of the two-dimensional image 67.

As described above, the CPU 57 of the console 14 comprises the acquisition unit 75, the extraction unit 76, the demarcation unit 77, the determination unit 78, and the display control unit 79. The acquisition unit 75 acquires the two-dimensional image 67 obtained by imaging the subject H who undergoes the radiography via the camera 20. The extraction unit 76 extracts the portion of interest POI of the subject H included in the two-dimensional image 67. The demarcation unit 77 demarcates the imaging region IR to be imaged by the radiography in the two-dimensional image 67 based on the portion of interest POI. The determination unit 78 determines whether or not the imaging preparation state before the radiography is appropriate based on the imaging region IR. The display control unit 79 outputs the information corresponding to the determination result 87 by displaying the circle mark 107, the messages 108 and 110 to 112, and the exclamation mark 109 in the display region 103 of the information display screen 88.

In JP6548713B, the three-dimensional information is acquired in addition to the two-dimensional image 67, and the portion of interest POI is extracted from the three-dimensional information in addition to the two-dimensional image 67. On the other hand, in the technology of the present disclosure, the portion of interest POI is extracted from only the two-dimensional image 67 without using the three-dimensional information. Therefore, the technology of the present disclosure does not require the cost and time and effort for extracting the portion of interest POI as compared with the technology disclosed in JP6548713B.

In the technology of the present disclosure, it is determined whether or not the imaging preparation state is appropriate based on the imaging region IR. Therefore, it is possible to supplement the determination of whether or not the imaging preparation state is appropriate, which is insufficient to be determined based on the portion of interest described in JP6548713B. Therefore, with the technology of the present disclosure, it is possible to determine whether or not the imaging preparation state is appropriate in a simpler and more sufficient manner.

In a case in which the imaging region IR includes the unnecessary part for the radiography, the determination unit 78 determines that the imaging preparation state is not appropriate. Therefore, it is possible to prevent the subject H from being exposed to unnecessary radiation due to the imaging failure caused by the radiography being performed while the unnecessary part is included in the imaging region IR. In addition, the probability of obtaining the radiation image 66 suitable for the diagnosis is increased.

The determination unit 78 determines that the imaging preparation state is not appropriate in a case in which the deviation between the center IRC of the imaging region IR and the irradiation center RC of the radiation R is equal to or greater than the preset threshold value. Therefore, it is possible to prevent the subject H from being exposed to unnecessary radiation due to the imaging failure caused by the radiography being performed while the center IRC of the imaging region IR and the irradiation center RC of the radiation R are deviated from each other. In addition, the probability of obtaining the radiation image 66 suitable for the diagnosis is increased.

The display control unit 79 performs control of displaying, on the display 40, the deviation amount 114 between the center IRC of the imaging region IR and the irradiation center RC of the radiation R in a real space by displaying the dialog box 113 in the display region 102 of the information display screen 88. Therefore, the operator OP can easily grasp the deviation amount 114 between the center IRC of the imaging region IR and the irradiation center RC of the radiation R in the real space.

The display control unit 79 displays the dialog box 113 in the display region 102 of the information display screen 88, to perform control of displaying, on the display 40, the adjustment amounts 115 of the positions of the radiation source 16 and the electronic cassette 13, the adjustment amounts 115 being for reducing the deviation between the center IRC of the imaging region IR and the irradiation center RC of the radiation R. Therefore, the operator OP can easily grasp the adjustment amounts 115 of the positions of the radiation source 16 and the electronic cassette 13, the adjustment amounts 115 being for reducing the deviation between the center IRC of the imaging region IR and the irradiation center RC of the radiation R.

The display control unit 79 performs control of displaying, on the display 40, the two-dimensional image 67 on which the frame 105 indicating the imaging region IR is superimposed. Therefore, the operator OP can visually recognize the imaging region IR through the display 40 and can give an accurate instruction to the subject H through the microphone 28.

The determination unit 78 detects the degree of inclination of the posture of the subject H based on the portion of interest POI. In a case in which the degree of inclination is out of the preset range, the determination unit 78 determines that the imaging preparation state is not appropriate. Therefore, it is possible to prevent the subject H from being exposed to unnecessary radiation due to the imaging failure caused by the radiography being performed while the posture of the subject H is greatly inclined. In addition, the probability of obtaining the radiation image 66 suitable for the diagnosis is increased.

### (Modification Example 1)

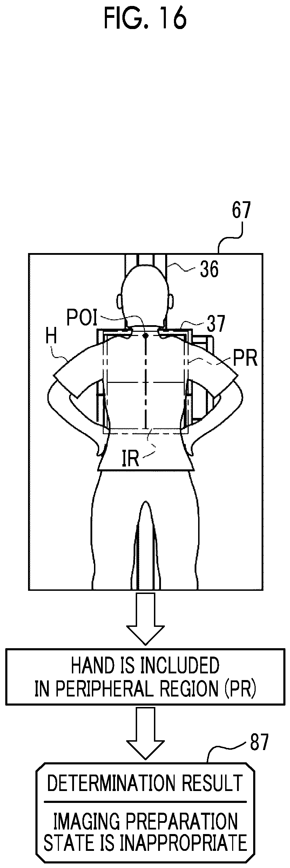

In Figs. 8A and 8B, it is determined whether or not the imaging preparation state is appropriate, depending on whether or not the hand is included in the imaging region IR, but the present disclosure is not limited to this. As shown in Fig. 16 as an example, it may be determined whether or not the imaging preparation state is appropriate, depending on whether or not the hand is included in a preset peripheral region PR of the imaging region IR, instead of the imaging region IR. The peripheral region PR is a rectangular region having the same center as the imaging region IR, and has a size, for example, 1.1 times the size of the imaging region IR. Fig. 16 shows a case in which the hand is included in the peripheral region PR, and the determination unit 78 determines that the imaging preparation state is not appropriate. Since the condition in which the imaging preparation state is determined to be appropriate is more severe than a case of the imaging region IR, it is possible to more reliably prevent the radiography from being performed while the unnecessary part is included in the imaging region IR, the imaging failure from occurring, and the subject H from being exposed to unnecessary radiation. Further, there is a higher possibility of obtaining the radiation image 66 suitable for the diagnosis.

It should be noted that the operator OP may display a warning in a stepwise manner. Specifically, in a case in which the hand is included in the peripheral region PR but is not included in the imaging region IR, a message such as "The imaging preparation state is appropriate, but the hand is included in the peripheral region, so please be careful." is displayed in the display region 103 together with the circle mark 107. In a case in which the hand is included in the imaging region IR, the exclamation mark 109 and the message 110 are displayed in the display region 103. In this manner, the operator OP can prompt the subject H to pay attention before the hand is included in the imaging region IR.

### (Modification Example 2)

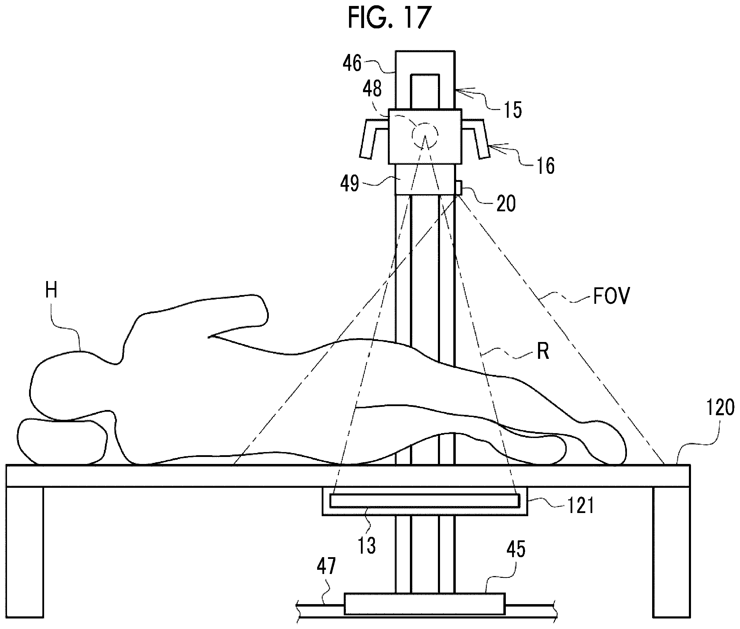

A case of the chest portion upright front surface imaging has been described as an example so far, but in Modification Example 2, a case of knee decubitus side surface imaging will be described. In this case, for example, as shown in Fig. 17, the subject H lies down on a decubitus imaging table 120 with a knee, here, a left knee imaged by the radiography down. The operator OP uses an auxiliary tool, such as a cushion and/or a table, as necessary in order to maintain the posture of the subject H. The left knee is an example of an "imaging part to be imaged by the radiography" according to the technology of the present disclosure.

The decubitus imaging table 120 is installed in the radiography room 25 together with the upright imaging table 12 and the like. The decubitus imaging table 120 includes a holder 121 that holds the electronic cassette 13 inside. The holder 121 can be moved parallel along a long side of the decubitus imaging table 120.

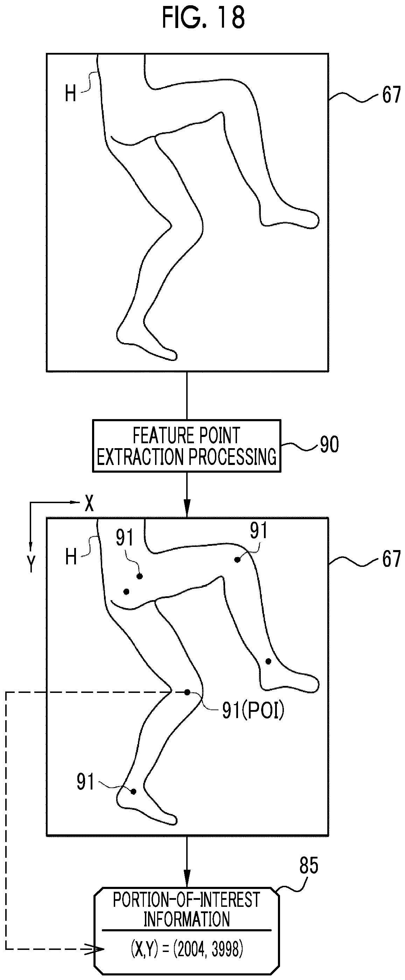

As shown in Fig. 18 as an example, in the two-dimensional image 67 in this case, a side surface of the subject H lying on the decubitus imaging table 120 from the waist to the toes (a part of the upper body and the entire lower body) is shown. The extraction unit 76 performs the feature point extraction processing 90 on the two-dimensional image 67 and extracts the right and left hip joint points, the right and left knee joint points, and the right and left ankle joint points, as the feature points 91. The ankle j oint point is the connection point between the shinbone and a talus. The extraction unit 76 extracts the left knee j oint point among the feature points 91, as the portion of interest POI. Therefore, the left knee j oint point is also the feature point 91 and is also an example of a "portion of interest" according to the technology of the present disclosure. In addition, the feature point extraction processing 90 is also the portion-of-interest extraction processing 92. The extraction unit 76 outputs the XY coordinates of the left knee joint point to the demarcation unit 77 as the portion-of-interest information 85. It should be noted that, in this case, the X-axis of the two-dimensional image 67 is parallel to a short side of the decubitus imaging table 120, and the Y-axis is parallel to a long side of the decubitus imaging table 120.

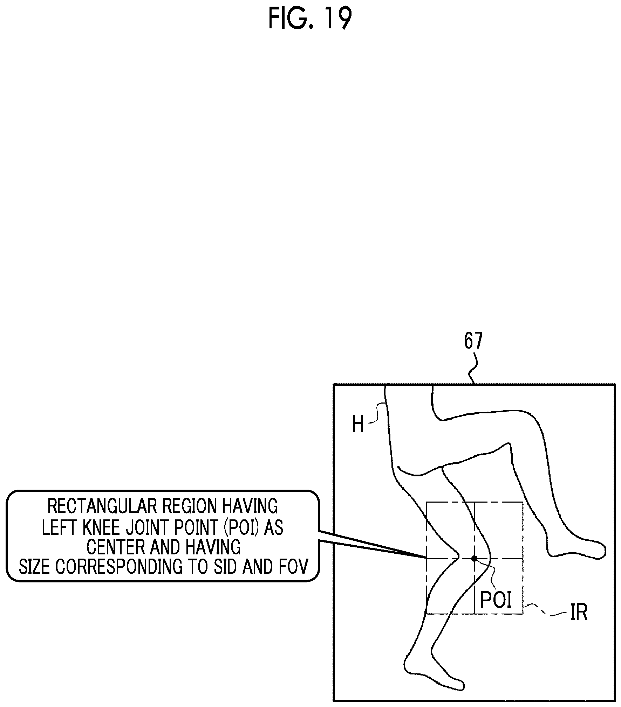

As shown in Fig. 19 as an example, the demarcation unit 77 demarcates a rectangular region having the left knee joint point as the center and having a size corresponding to the SID and the field of view FOV of the camera 20, as the imaging region IR. Although not shown, the demarcation unit 77 outputs the XY coordinates of the four vertices of the imaging region IR to the determination unit 78 and the like as the imaging region information 86.

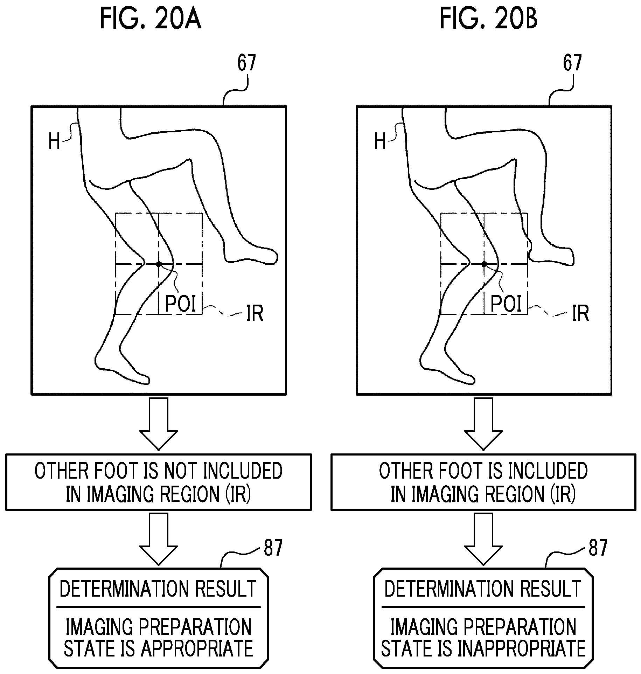

As shown in Figs. 20A and 20B as an example, the determination unit 78 determines whether or not the imaging preparation state is appropriate, depending on whether or not the other foot, here, the right foot of the subject H is included in the imaging region IR. Whether or not the other foot of the subject H is included in the imaging region IR is determined, for example, depending on whether or not a distance between the ankle joint point and the imaging region IR is less than the threshold value. As shown in Fig. 20A, in a case in which the other foot is not included in the imaging region IR, the determination unit 78 determines that the imaging preparation state is appropriate, and outputs the determination result 87 indicating that the imaging preparation state is appropriate. On the other hand, as shown in Fig. 20B, in a case in which the other foot is included in the imaging region IR, the determination unit 78 determines that the imaging preparation state is not appropriate, and outputs the determination result 87 indicating that the imaging preparation state is not appropriate. The other foot is an example of an "unnecessary part for the radiography" according to the technology of the present disclosure.

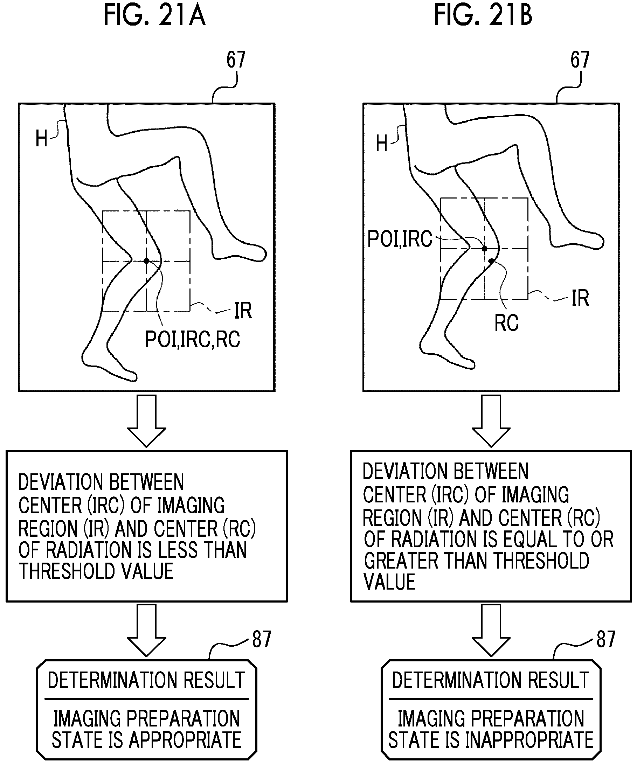

As shown in Figs. 21A and 21B as an example, the determination unit 78 determines whether or not the imaging preparation state is appropriate based on the deviation (distance) between the center IRC of the imaging region IR and the irradiation center RC of the radiation R. As shown in Fig. 21A, in a case in which the deviation between the center IRC of the imaging region IR and the irradiation center RC of the radiation R is less than the threshold value, the determination unit 78 determines that the imaging preparation state is appropriate, and outputs the determination result 87 indicating that the imaging preparation state is appropriate. On the other hand, as shown in Fig. 21B, in a case in which the deviation between the center IRC of the imaging region IR and the irradiation center RC of the radiation R is equal to or greater than the threshold value, the determination unit 78 determines that the imaging preparation state is not appropriate, and outputs the determination result 87 indicating that the imaging preparation state is not appropriate. It should be noted that the threshold value in this case is, for example, 5 cm.

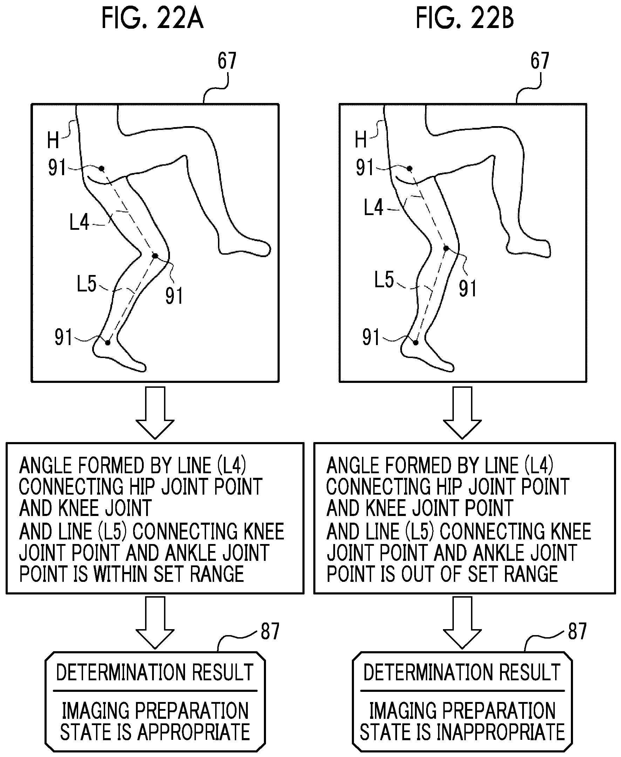

As an example, as shown in Figs. 22A and 22B, the determination unit 78 detects a bending angle of the left knee based on the left hip joint point, the left knee joint point, and the left ankle joint point. More specifically, the determination unit 78 detects an angle formed by a line L4 connecting the left hip joint point and the left knee j oint point and a line L5 connecting the left knee joint point and the left ankle joint point, as the bending angle of the left knee. Therefore, similarly to the left knee joint point, the left hip joint point and the left ankle joint point are also the feature points 91 and are also examples of a "portion of interest" according to the technology of the present disclosure.

The determination unit 78 determines whether or not the imaging preparation state is appropriate based on the bending angle of the left knee. As shown in Fig. 22A, in a case in which the angle formed by the line L4 connecting the left hip joint point and the left knee joint point and the line L5 connecting the left knee joint point and the left ankle joint point is within the set range, the determination unit 78 determines that the imaging preparation state is appropriate, and outputs the determination result 87 indicating that the imaging preparation state is appropriate. On the other hand, as shown in Fig. 22B, in a case in which the angle formed by the line L4 connecting the left hip joint point and the left knee joint point and the line L5 connecting the left knee joint point and the left ankle joint point is out of the set range, the determination unit 78 determines that the imaging preparation state is not appropriate, and outputs the determination result 87 indicating that the imaging preparation state is not appropriate. It should be noted that the set range in this case is, for example, equal to or greater than 125° and equal to or less 145° (135° ± 10°).

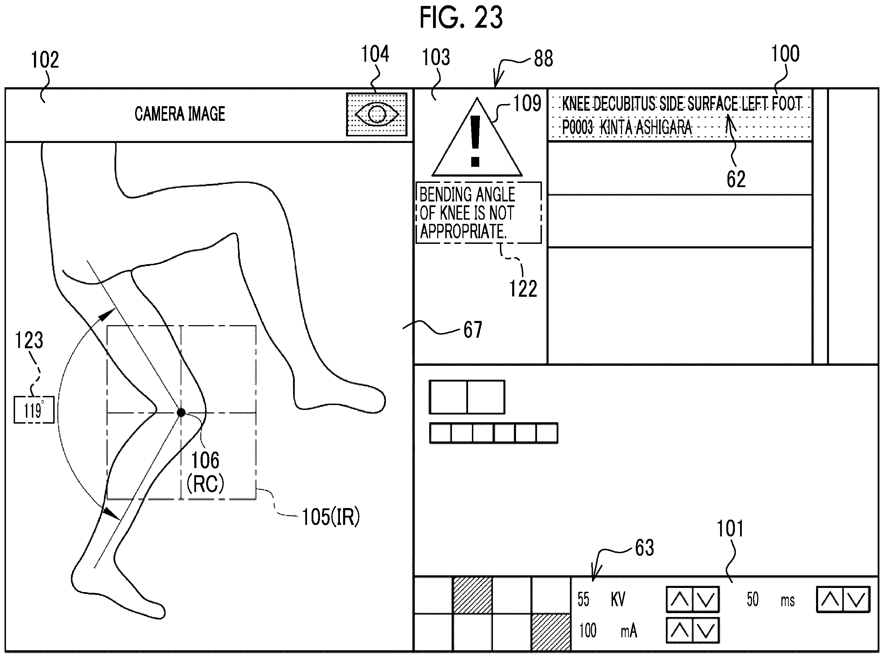

Fig. 23 shows the information display screen 88 in a case in which the determination result 87 indicates that the angle formed by the line L4 connecting the left hip joint point and the left knee joint point and the line L5 connecting the left knee joint point and the left ankle joint point is out of the set range and that the imaging preparation state is not appropriate (in a case of Fig. 22B), in the determinations shown in Figs. 22A and 22B. In this case, the exclamation mark 109 and a message 122 indicating that the bending angle of the knee is not appropriate are displayed in the display region 103. In addition, a numerical value 123 indicating the bending angle of the knee is displayed on the two-dimensional image 67 in the display region 102. The operator OP instructs the subject H to correct the bending angle of the left knee by making an announcement to the speaker 27 of the radiography room 25 via the microphone 28.

As described above, the determination unit 78 detects the angle of the imaging part to be imaged by the radiography based on the portion of interest POI. The determination unit 78 determines that the imaging preparation state is not appropriate in a case in which the angle of the imaging part is out of the preset range. Therefore, it is possible to prevent the subject H from being exposed to unnecessary radiation due to the imaging failure caused by the radiography being performed while the angle of the imaging part is out of the set range. In addition, the probability of obtaining the radiation image 66 suitable for the diagnosis is increased.

### (Modification Example 3)

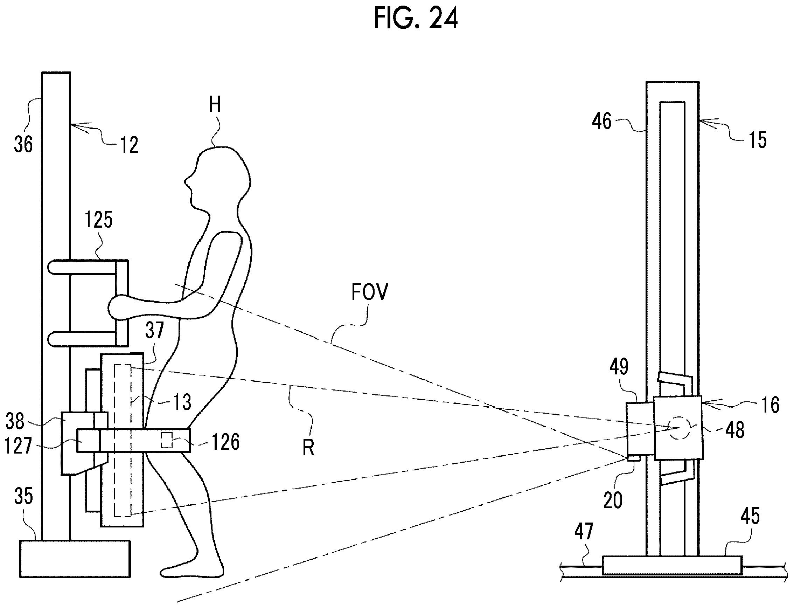

In Modification Example 3, a case of the knee upright front surface imaging using the Rosenberg method will be described. In this case, as shown in Fig. 24 as an example, the subject H stands in front of the upright imaging table 12 in a state in which both knees are bent toward the electronic cassette 13. The operator OP attaches a handrail 125 to the upright imaging table 12 as necessary, in order to maintain the posture of the subject H. The operator OP rotates the radiation source 16 from the horizontal direction to the toe side by approximately 10° in order to cause the radiation R to be obliquely incident on both knees from the upper part. Further, the operator OP attaches an arm 127 on which the camera 126 that mainly images both knees is disposed at the distal end to the connecting part 38. Both knees are examples of an "imaging part to be imaged by the radiography" according to the technology of the present disclosure.

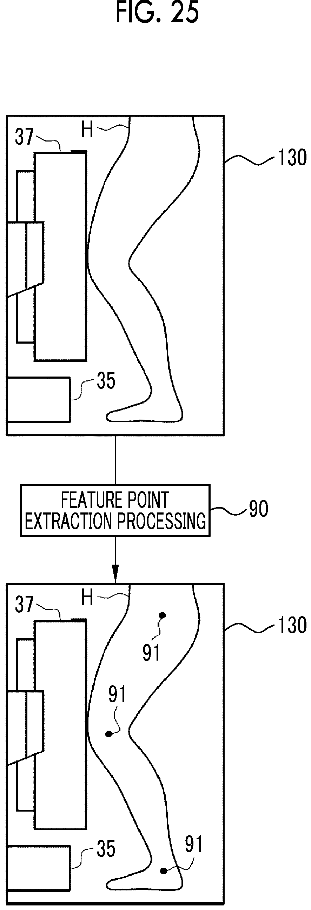

In this case, the acquisition unit 75 acquires a two-dimensional image 130 (see Fig. 25) captured by the camera 126 in addition to the two-dimensional image 67 captured by the camera 20. Although not shown, the extraction unit 76 performs the feature point extraction processing 90 on the two-dimensional image 67, to extract the right and left hip joint points, the right and left knee joint points, and the right and left ankle joint points as the feature points 91. Although not shown, the demarcation unit 77 demarcates, for example, a rectangular region having a midpoint of a line connecting the right and left knee joint points as the center and having a size according to the SID and the FOV of the camera 20, as the imaging region IR.

As shown in Fig. 25 as an example, the two-dimensional image 130 shows the side surface of the subject H who stands in front of the upright imaging table 12 from the waist to the toes (a part of the upper body and the entire lower body) in a state in which both knees are bent. The extraction unit 76 also performs the feature point extraction processing 90 on the two-dimensional image 130 to extract the left hip joint point, the left knee joint point, and the left ankle joint point as the feature points 91.

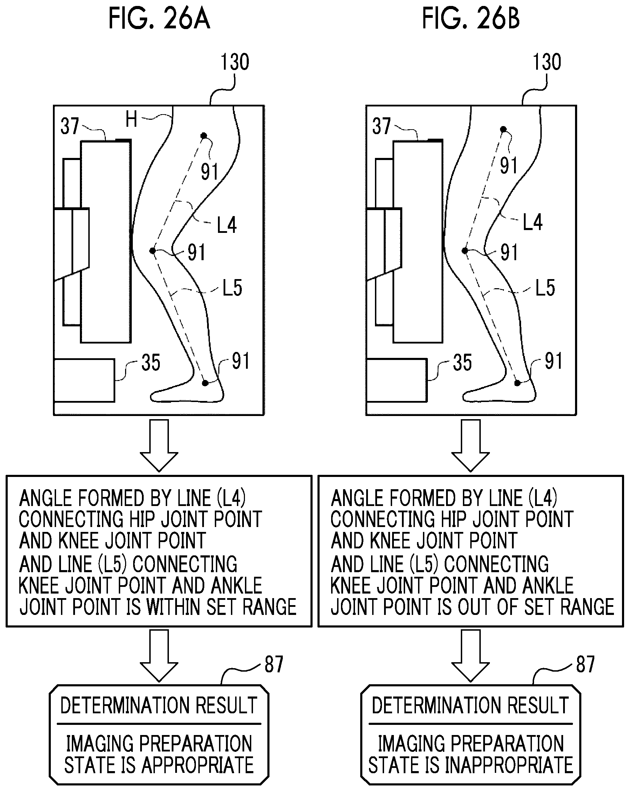

As shown in Figs. 26A and 26B as an example, the determination unit 78 determines whether or not the imaging preparation state is appropriate, based on the bending angle of the left knee, in the same manner as in a case of Modification Example 2. As shown in Fig. 26A, in a case in which the angle formed by the line L4 connecting the left hip joint point and the left knee j oint point and the line L5 connecting the left knee j oint point and the left ankle joint point is within the set range, the determination unit 78 determines that the imaging preparation state is appropriate, and outputs the determination result 87 indicating that the imaging preparation state is appropriate. On the other hand, as shown in Fig. 26B, in a case in which the angle formed by the line L4 connecting the left hip joint point and the left knee joint point and the line L5 connecting the left knee joint point and the left ankle joint point is out of the set range, the determination unit 78 determines that the imaging preparation state is not appropriate, and outputs the determination result 87 indicating that the imaging preparation state is not appropriate. It should be noted that the set range in this case is, for example, equal to or greater than 115° and equal to or less 135° (125° ± 10°).

Although not shown, the two-dimensional image 67 and the two-dimensional image 130 are displayed in the display region 102 of the information display screen 88 in a switchable manner. In a case in which the determination result 87 indicates that the angle formed by the line L4 connecting the left hip joint point and the left knee joint point and the line L5 connecting the left knee joint point and the left ankle joint point is out of the set range and that the imaging preparation state is not appropriate (in a case of Fig. 26B), as in the information display screen 88 shown in Fig. 23 of Modification Example 2, the exclamation mark 109 and the message 122 indicating that the bending angle of the knee is not appropriate are displayed in the display region 103. In addition, the numerical value 123 indicating the bending angle of the knee is displayed on the two-dimensional image 130 in the display region 102. The operator OP instructs the subject H to correct the bending angle of the both knees by making an announcement to the speaker 27 of the radiography room 25 via the microphone 28.

As described above, the camera that images the subject H who undergoes the radiography is not limited to one camera 20 provided in the radiation source 16. In addition, also in Modification Example 3, it is possible to prevent the subject H from being exposed to unnecessary radiation due to the imaging failure caused by the radiography being performed while the angle of the imaging part is out of the set range. In addition, the probability of obtaining the radiation image 66 suitable for the diagnosis is increased. It should be noted that the handrail 125 and the arm 127 in which the camera 126 is disposed at the distal end may be attached to the upright imaging table 12 in advance.

### (Modification Example 4)

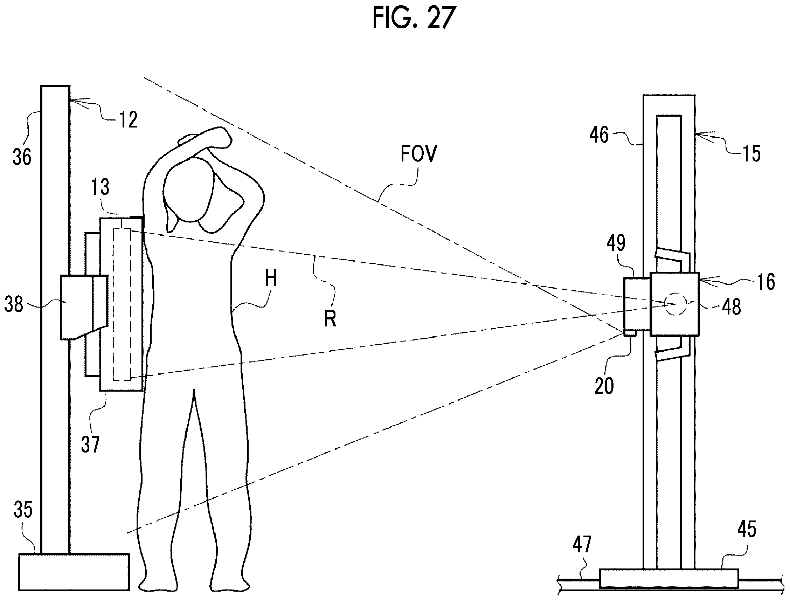

In Modification Example 4, a case of the chest portion upright side surface imaging will be described. In this case, as shown in Fig. 27 as an example, the subj ect H stands in front of the upright imaging table 12 in a state in which both arms are raised and, for example, the right flank is in close contact with the holder 37, and the coronal plane is perpendicular to the detection surface of the radiation R of the electronic cassette 13. The operator OP attaches a handrail (not shown) to the upright imaging table 12 as necessary, in order to maintain the posture of the subject H. It should be noted that the handrail may be attached to the upright imaging table 12 in advance.

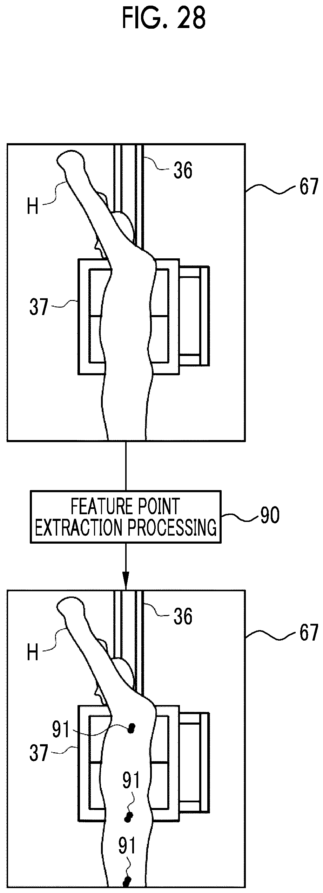

As shown in Fig. 28 as an example, the two-dimensional image 67 in this case shows the side surface of the subject H who stands in front of the upright imaging table 12 from the top of the head portion to below the knee (the entire upper body and a part of the lower body) in a state in which both arms are raised. The extraction unit 76 performs the feature point extraction processing 90 on the two-dimensional image 67 and extracts the right and left shoulder joint points, the right and left hip joint points, and the right and left knee joint points, as the feature points 91. In addition, although not shown, the extraction unit 76 extracts the prominent vertebra point from the right and left shoulder joint points and the right and left hip joint points, as in a case of the chest portion upright front surface imaging. Although not shown, the demarcation unit 77 demarcates a rectangular region having the prominent vertebra point as the center of the upper side and having a size corresponding to the SID and the FOV of the camera 20, as the imaging region IR, as in a case of the chest portion upright front surface imaging.

As shown in Figs. 29A and 29B as an example, the determination unit 78 detects the degree of inclination of the posture of the subject H based on the right and left shoulder joint points, the right and left hip joint points, and the right and left knee joint points. More specifically, the determination unit 78 detects a distance between the right and left shoulder joint points, a distance between the right and left hip joint points, and a distance between the right and left knee joint points, as the degrees of inclination of the posture of the subject H.

The determination unit 78 determines whether or not the imaging preparation state is appropriate, depending on the degree of inclination of the posture of the subject H. As shown in Fig. 29A, in a case in which the distance between the right and left shoulder joint points, the distance between the right and left hip joint points, and the distance between the right and left knee joint points are less than a threshold value, the determination unit 78 determines that the imaging preparation state is appropriate, and outputs the determination result 87 indicating that the imaging preparation state is appropriate. On the other hand, as shown in Fig. 29B, in a case in which any one of the distance between the right and left shoulder joint points, the distance between the right and left hip joint points, or the distance between the right and left knee joint points is equal to or greater than the threshold value, the determination unit 78 determines that the imaging preparation state is not appropriate, and outputs the determination result 87 indicating that the imaging preparation state is not appropriate. It should be noted that the threshold value in this case is, for example, 5 cm.

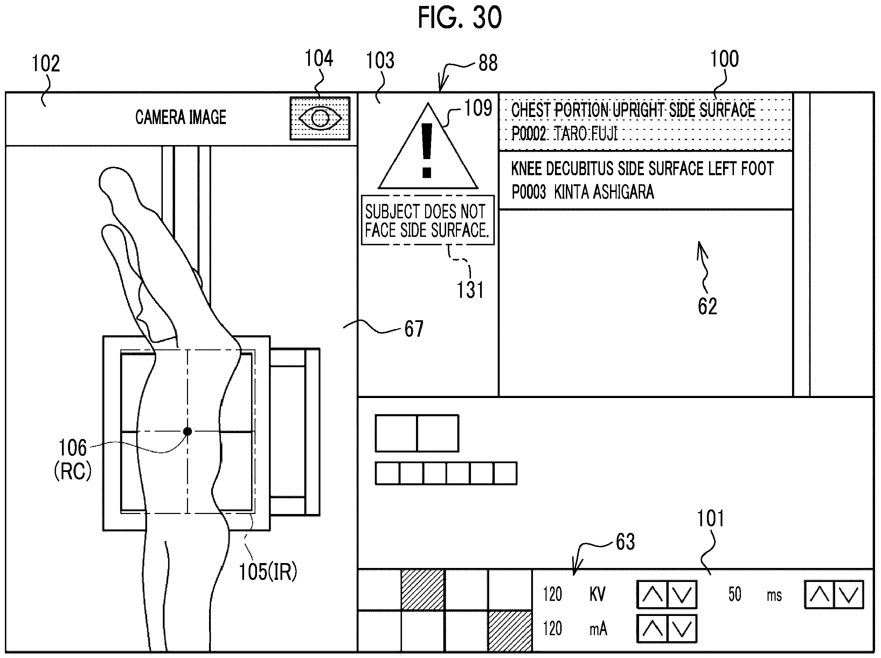

Fig. 30 shows the information display screen 88 in a case in which the determination result 87 indicates that any one of the distance between the right and left shoulder joint points, the distance between the right and left hip joint points, or the distance between the right and left knee joint points is equal to or greater than the threshold value and that the imaging preparation state is not appropriate (in a case of Fig. 29B), in the determinations shown in Figs. 29A and 29B. In this case, the exclamation mark 109 and a message 131 indicating that the subject H does not accurately face the side surface are displayed in the display region 103. The operator OP instructs the subject H to accurately face the side surface by making an announcement to the speaker 27 of the radiography room 25 via the microphone 28.

Also in Modification Example 4, it is possible to prevent the subject H from being exposed to unnecessary radiation due to the imaging failure caused by the radiography being performed while the posture of the subject H is greatly inclined. In addition, the probability of obtaining the radiation image 66 suitable for the diagnosis is increased. It should be noted that, in Modification Example 3 of the knee upright side surface imaging using the Rosenberg method, similarly, the degree of inclination of the posture of the subject H may be detected based on the distance between the right and left hip joint points, the distance between the right and left knee joint points, and the distance between the right and left ankle joint points, and it may be determined whether or not the imaging preparation state is appropriate, based on the degree of inclination.

### (Modification Example 5)

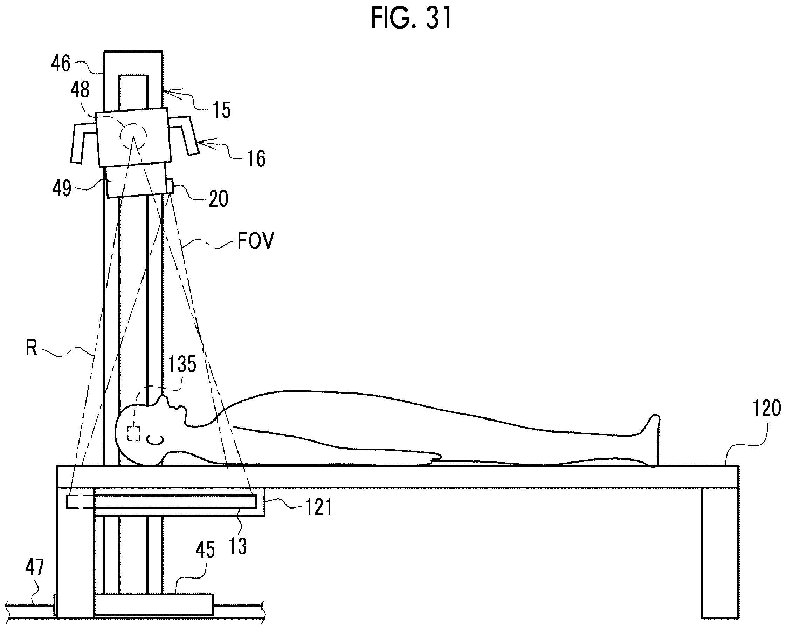

In Modification Example 5, a case of the head portion decubitus side surface imaging using the Towne method will be described. In this case, the subject H is, for example, as shown in Fig. 31, in the decubitus imaging table 120. The operator OP rotates the radiation source 16 from the vertical direction to the toe side by about 30° in order to cause the radiation R to be obliquely incident on the top of the head portion. In addition, the operator OP attaches the camera 135 that mainly images the head portion to the support column 46 of the radiation source table 15. The head portion is an example of an "imaging part to be imaged by the radiography" according to the technology of the present disclosure. It should be noted that the camera 135 may be attached to the radiation source table 15 in advance.

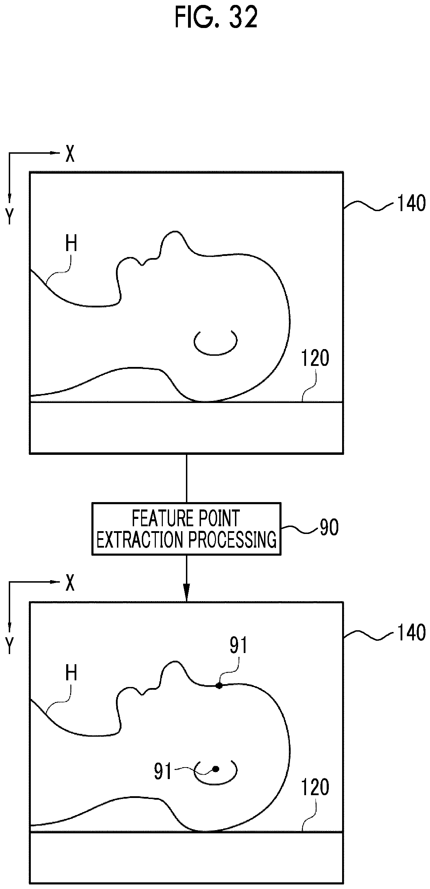

In this case, the acquisition unit 75 acquires a two-dimensional image 140 (see Fig. 32) captured by the camera 135 in addition to the two-dimensional image 67 captured by the camera 20. Although not shown, the extraction unit 76 extracts the right and left orbital points and the right and left external auditory canal points as the feature points 91 by performing the feature point extraction processing 90 on the two-dimensional image 67. As is well known, an orbit is a depression in which an eyeball is accommodated, and the orbit point is a center point of the depression.

Although not shown, the demarcation unit 77 demarcates, for example, a rectangular region having a midpoint of a line connecting the right and left external auditory canal points as the center and having a size according to the SID and the FOV of the camera 20, as the imaging region IR.

As shown in Fig. 32 as an example, the two-dimensional image 140 shows the side surface of the subject H lying down on the decubitus imaging table 120 from the top of the head portion to the shoulder. The extraction unit 76 also performs the feature point extraction processing 90 on the two-dimensional image 140 to extract the left orbit point and the left external auditory canal point as the feature points 91. It should be noted that the X-axis of the two-dimensional image 140 is parallel to the long side of the decubitus imaging table 120, and the Y-axis is parallel to the vertical direction.

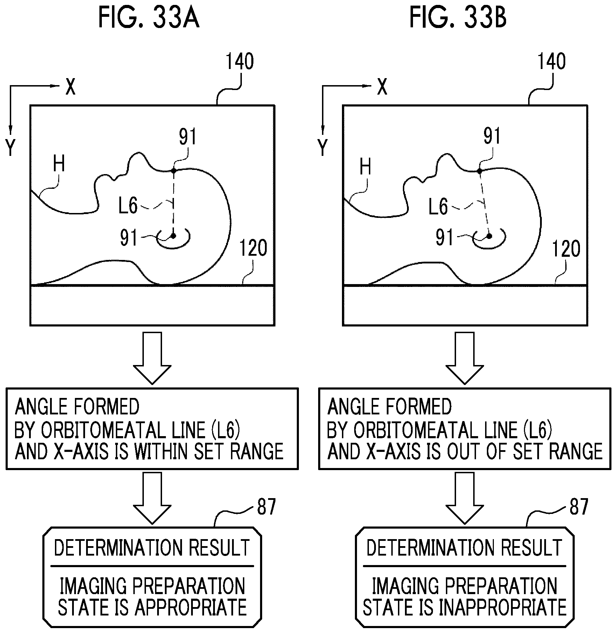

As shown in Figs. 33A and 33B as an example, the determination unit 78 detects an inclination angle of the head portion based on the left orbit point and the left external auditory canal point. More specifically, the determination unit 78 detects an angle formed by a line (orbitomeatal line, or called an orbitomeatal base (OM) line) L6 connecting the left orbit point and the left external auditory canal point, and the X-axis (top surface of the decubitus imaging table 120) as the inclination angle of the head portion. Therefore, the left orbit point and the left external auditory canal point are the feature points 91 and are also examples of a "portion of interest" according to the technology of the present disclosure. In addition, the feature point extraction processing 90 shown in Fig. 32 is also the portion-of-interest extraction processing 92.

The determination unit 78 determines whether or not the imaging preparation state is appropriate, depending on the inclination angle of the head portion. As shown in Fig. 33A, in a case in which the angle formed by the line L6 connecting the left orbit point and the left external auditory canal point, and the X-axis is within a set range, the determination unit 78 determines that the imaging preparation state is appropriate, and outputs the determination result 87 indicating that the imaging preparation state is appropriate. On the other hand, as shown in Fig. 33B, in a case in which the angle formed by the line L6 connecting the left orbit point and the left external auditory canal point, and the X-axis is out of the set range, the determination unit 78 determines that the imaging preparation state is not appropriate, and outputs the determination result 87 indicating that the imaging preparation state is not appropriate. It should be noted that the set range in this case is, for example, equal to or greater than 85° and equal to or less 95° (90 ± 5°).

Although not shown, the two-dimensional image 67 and the two-dimensional image 140 are displayed in the display region 102 of the information display screen 88 in a switchable manner. In a case in which the determination result 87 indicates that the angle formed by the line L6 connecting the left orbit point and the left external auditory canal point, and the X-axis is out of the set range and that the imaging preparation state is not appropriate (in a case of Fig. 33B), the exclamation mark 109 and a message indicating that the inclination angle of the head portion is not appropriate are displayed in the display region 103. In addition, a numerical value indicating the inclination angle of the head portion is displayed on the two-dimensional image 140 of the display region 102. The operator OP instructs the subject H to correct the inclination angle of the head portion by making an announcement to the speaker 27 of the radiography room 25 via the microphone 28.

Also in Modification Example 5, it is possible to prevent the subject H from being exposed to unnecessary radiation due to the imaging failure caused by the radiography being performed while the angle of the imaging part is out of the set range. In addition, the probability of obtaining the radiation image 66 suitable for the diagnosis is increased.

### (Modification Example 6)

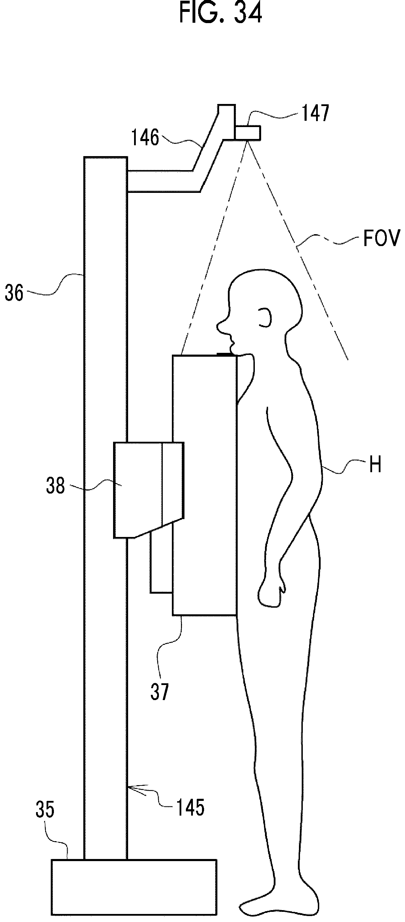

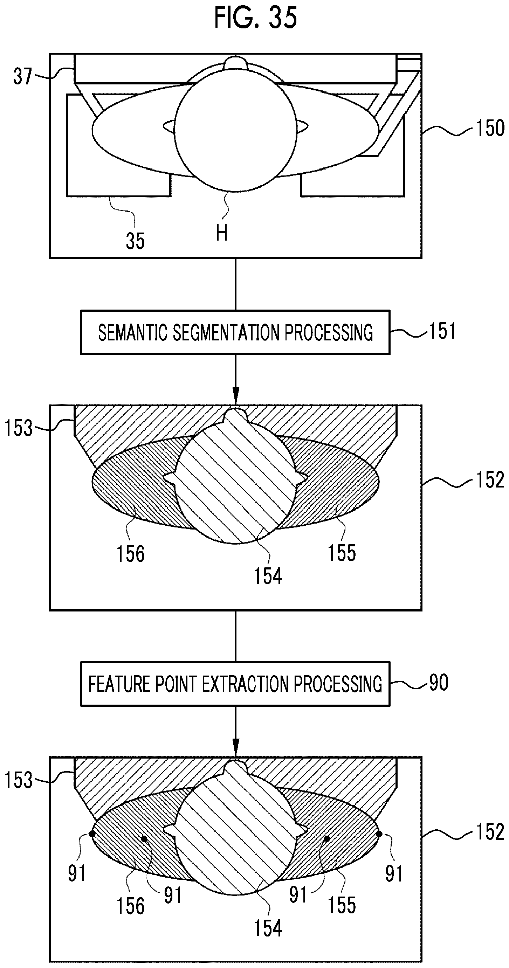

In Modification Example 6, a case in which the chest portion upright front surface imaging is performed using an upright imaging table 145 shown in Fig. 34 as an example will be described. The basic configuration of the upright imaging table 145 is the same as that of the upright imaging table 12, but which is different from the upright imaging table 12 in that the camera 147 is attached to the upper end part of the support column 36 via the arm 146. In this case, the acquisition unit 75 acquires a two-dimensional image 150 (see Fig. 35) captured by the camera 147 in addition to the two-dimensional image 67 captured by the camera 20.

As shown in Fig. 35 as an example, the two-dimensional image 150 shows the holder 37 of the upright imaging table 12 and the like, and the subject H who stands in front of the upright imaging table 12 from above. The extraction unit 76 performs semantic segmentation processing 151 on the two-dimensional image 150 to generate a segmentation image 152 from the two-dimensional image 150. The semantic segmentation processing 151 is processing of identifying an obj ect shown in the image in a pixel unit by using a machine learning model such as a convolutional neural network.

The segmentation image 152 is an image in which a region 153 of the holder 37, a region 154 of the head portion of the subject H, a region 155 of the right shoulder portion, and a region 156 of the left shoulder portion are identified. The extraction unit 76 performs the feature point extraction processing 90 on the segmentation image 152 to extract the right and left shoulder joint points and the right and left elbow joint points, as the feature points 91. The right and left shoulder joint points are, for example, the centroids of the region 155 of the right shoulder portion and the region 156 of the left shoulder portion, respectively. The right and left elbow joint points are, for example, the distal end points of the region 155 of the right shoulder portion and the region 156 of the left shoulder portion, respectively.

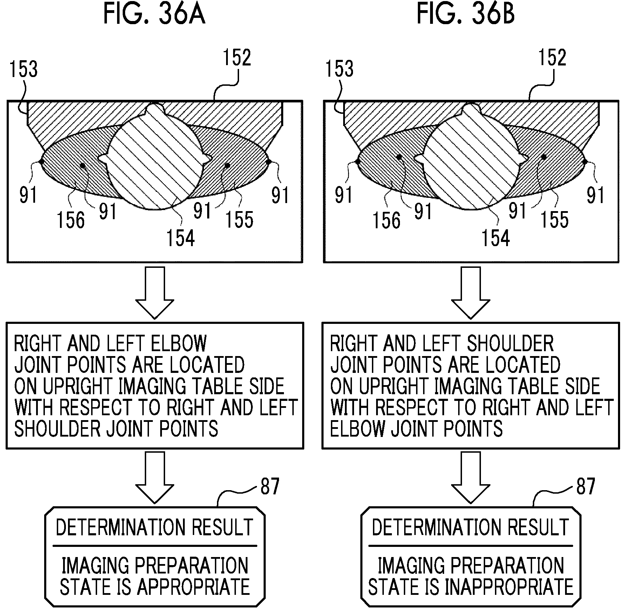

As shown in Figs. 36A and 36B as an example, the determination unit 78 detects a degree of opening of the shoulder blade of the subj ect H based on the right and left shoulder j oint points and the right and left elbow joint points. Specifically, the determination unit 78 detects the degree of opening of the shoulder blade of the subject H based on which of the right and left shoulder j oint points or the right and left elbow j oint points is located on the upright imaging table 145 side.

The determination unit 78 determines whether or not the imaging preparation state is appropriate, depending on the degree of opening of the shoulder blade of the subject H. As shown in Fig. 36A, in a case in which the right and left elbow joint points are located on the upright imaging table 145 side with respect to the right and left shoulder joint points, the determination unit 78 determines that the imaging preparation state is appropriate, and outputs the determination result 87 indicating that the imaging preparation state is appropriate. On the other hand, as shown in Fig. 36B, in a case in which the right and left shoulder joint points are located on the upright imaging table 145 side with respect to the right and left elbow j oint points, the determination unit 78 determines that the imaging preparation state is not appropriate, and outputs the determination result 87 indicating that the imaging preparation state is not appropriate.

Although not shown, the two-dimensional image 67 and the two-dimensional image 150 are displayed in the display region 102 of the information display screen 88 in a switchable manner. In a case in which the determination result 87 indicates that the right and left shoulder joint points are located on the upright imaging table 145 side with respect to the right and left elbow joint points and that the imaging preparation state is not appropriate (in a case of Fig. 36B), the exclamation mark 109 and a message indicating that the degree of opening of the shoulder blade is not appropriate are displayed in the display region 103. The operator OP instructs the subject H to protrude the elbow forward to open the shoulder blade by making an announcement to the speaker 27 of the radiography room 25 via the microphone 28.

In Modification Example 6, it is possible to prevent the subject H from being exposed to unnecessary radiation due to the imaging failure caused by the radiography being performed while the degree of opening of the shoulder blade of the subject H is insufficient. In addition, the probability of obtaining the radiation image 66 suitable for the diagnosis is increased.

It should be noted that the degree of opening of the shoulder blade of the subject H may be detected based on a distance between the region of the holder 37 and the right and left elbow joint points. Alternatively, the feature point extraction processing 90 may be performed on the two-dimensional image 150 without performing the semantic segmentation processing 151, the right and left shoulder joint points and the right and left elbow joint points may be extracted as the feature points 91, the degree of opening of the shoulder blade of the subject H may be detected based on the right and left shoulder joint points and the right and left elbow joint points, and it may be determined whether or not the imaging preparation state is appropriate, based on the degree of opening of the shoulder blade of the subject H.

### [Embodiment 2_1]

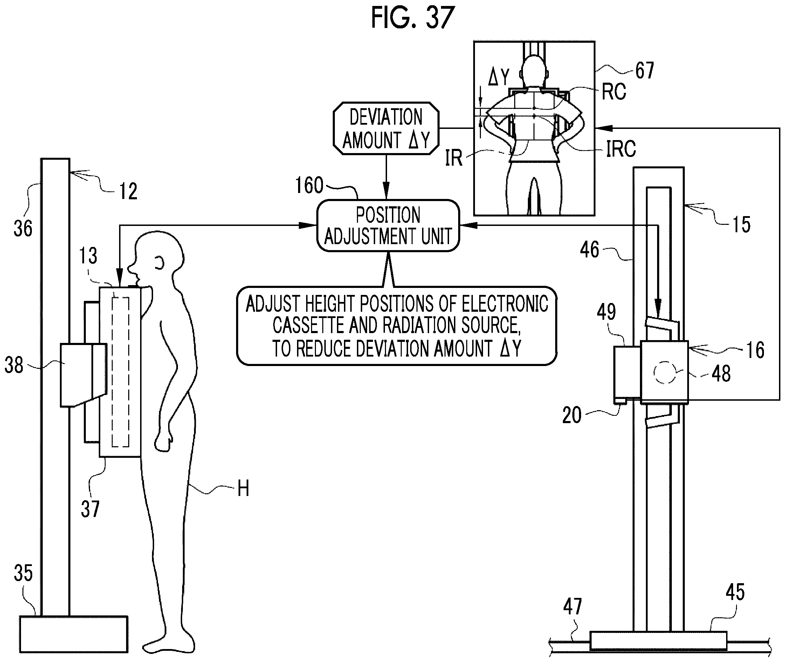

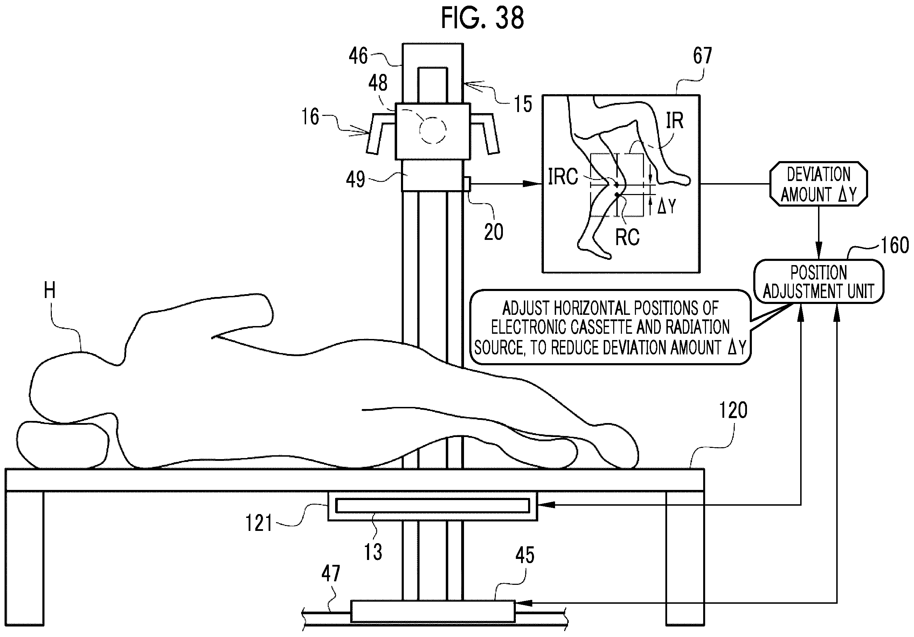

In Embodiment 1, in a case in which the deviation between the center IRC of the imaging region IR and the irradiation center RC of the radiation R is equal to or greater than a threshold value, the operator OP operates the console 14 to adjust the positions of the electronic cassette 13 (holder 37 or 121) and the radiation source 16 to reduce the deviation between the center IRC of the imaging region IR and the irradiation center RC of the radiation R, but the present disclosure is not limited to this. As shown in Figs. 37 and 38 as an example, as in Embodiment 2_1, the positions of the electronic cassette 13 and the radiation source 16 may be automatically adjusted without waiting for the operation of the console 14 by the operator OP, to reduce the deviation between the center IRC of the imaging region IR and the irradiation center RC of the radiation R. Here, the expression "to reduce" the deviation between the center IRC of the imaging region IR and the irradiation center RC of the radiation R includes a case in which the deviation between the center IRC of the imaging region IR and the irradiation center RC of the radiation R is set to zero, as well as a case in which the deviation between the center IRC of the imaging region IR and the irradiation center RC of the radiation R is set to less than the threshold value instead of zero.

In Fig. 37 and Fig. 38, the CPU 57 of the console 14 functions as a position adjustment unit 160 in addition to the processing units 75 to 79 (not shown) according to Embodiment 1. The position adjustment unit 160 acquires a deviation amount ΔY between the center IRC of the imaging region IR and the irradiation center RC of the radiation R in the Y-axis direction. The deviation amount ΔY can be calculated from the two-dimensional image 67 and the imaging region information 86 by the determination unit 78. In a case in which the deviation amount ΔY is equal to or greater than the threshold value, the position adjustment unit 160 performs control of adjusting the positions of the electronic cassette 13 and the radiation source 16 to reduce the deviation amount ΔY.

Fig. 37 shows a case of the chest portion upright front surface imaging. In this case, the position adjustment unit 160 first moves the holder 37 up and down along the support column 36 of the upright imaging table 12 in a direction of reducing the deviation amount ΔY to adjust the height position of the electronic cassette 13. Next, the position adjustment unit 160 moves the radiation source 16 up and down along the support column 46 of the radiation source table 15 to adjust the radiation source 16 to the height position corresponding to the height position of the electronic cassette 13. The position adjustment unit 160 performs these types of adjustment while grasping the height positions of the electronic cassette 13 and the radiation source 16 detected by the linear encoder. The function of automatically changing the height position of the radiation source 16 in conjunction with the change of the height position of the electronic cassette 13 is called an auto tracking function. It should be noted that, contrary to the auto tracking function, a reverse tracking function may be adopted in which the height position of the radiation source 16 is changed first and the height position of the electronic cassette 13 is changed in conjunction with the change of the height position of the radiation source 16.

Fig. 38 shows a case of the knee decubitus side surface imaging. In this case, the position adjustment unit 160 first moves the holder 121 along the long side of the decubitus imaging table 120 in a direction of reducing the deviation amount ΔY to adjust the horizontal position of the electronic cassette 13. Next, the position adjustment unit 160 moves the radiation source table 15 and the radiation source 16 along the rail 47 to adjust the radiation source 16 to the horizontal position corresponding to the horizontal position of the electronic cassette 13. The position adjustment unit 160 performs these types of adjustment while grasping the horizontal positions of the electronic cassette 13 and the radiation source 16 detected by the linear encoder and/or the potentiometer. It should be noted that, as in a case of Fig. 37, the horizontal position of the radiation source 16 may be changed first, and the horizontal position of the electronic cassette 13 may be changed in conjunction with the change of the horizontal position of the radiation source 16.

As described above, in Embodiment 2_1, the position adjustment unit 160 performs the control of adjusting the positions of the electronic cassette 13 and the radiation source 16, to reduce the deviation between the center IRC of the imaging region IR and the irradiation center RC of the radiation R. Therefore, it is possible to reduce the time and effort of the operation of the operator OP. In addition, it is possible to reduce the deviation between the center IRC of the imaging region IR and the irradiation center RC of the radiation R while avoiding the contact between the subject H and the operator OP.

Also in this case, as in Embodiment 1, the display control unit 79 displays the dialog box 113 in the display region 102 of the information display screen 88, and displays the deviation amount 114 between the center IRC of the imaging region IR and the irradiation center RC of the radiation R in a real space, which are calculated geometrically from the two-dimensional image 67 and the SID, and the adjustment amount 115 of the electronic cassette 13 and the radiation source 16 for setting the deviation between the center IRC of the imaging region IR and the irradiation center RC of the radiation R to zero.

It should be noted that the unit that detects the positions of the electronic cassette 13 and the radiation source 16 is not limited to the linear encoder as an example. The positions of the electronic cassette 13 and the radiation source 16 may be detected by a laser sensor or an ultrasound sensor.

### [Embodiment 2_2]

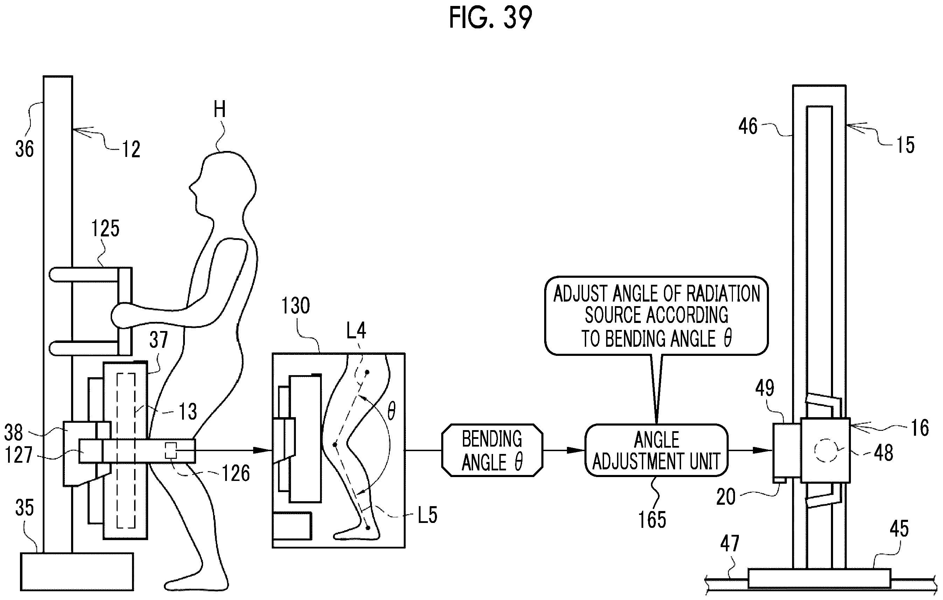

As shown in Fig. 39 as an example, Embodiment 2_2 is carried out in a case of the knee upright front surface imaging using the Rosenberg method. The CPU 57 of the console 14 functions as an angle adjustment unit 165 in addition to the processing units 75 to 79 (not shown) according to Embodiment 1. The angle adjustment unit 165 acquires a bending angle θ of the knee detected from the two-dimensional image 130 by the determination unit 78. The angle adjustment unit 165 performs control of adjusting an angle of the radiation source 16 with respect to the subject H according to the bending angle θ of the knee. First, the angle adjustment unit 165 calculates an angle of a supratibial articular surface with respect to the X-axis (floor surface of the radiography room 25) based on the bending angle θ of the knee. More specifically, the angle adjustment unit 165 uses a data table or an expression for converting the bending angle θ of the knee into the angle of the supratibial articular surface with respect to the X-axis. The angle adjustment unit 165 adjusts the rotation angle of the radiation source 16 to the calculated angle of the supratibial articular surface. That is, the radiation source 16 is rotated such that the radiation R is incident on the supratibial articular surface at an angle parallel to the supratibial articular surface.

As described above, in Embodiment 2_2, the control of adjusting the angle of the radiation source 16 with respect to the subject H according to the bending angle θ of the knee is performed. Therefore, it is possible to reduce the time and effort of the operation of the operator OP. In addition, the radiation source 16 can be set to the rotation angle corresponding to the bending angle θ of the knee while avoiding the contact between the subject H and the operator OP. It should be noted that the angle of the radiation source 16 with respect to the subject H according to the bending angle θ of the knee may be displayed on the information display screen 88.

### [Embodiment 3_1]

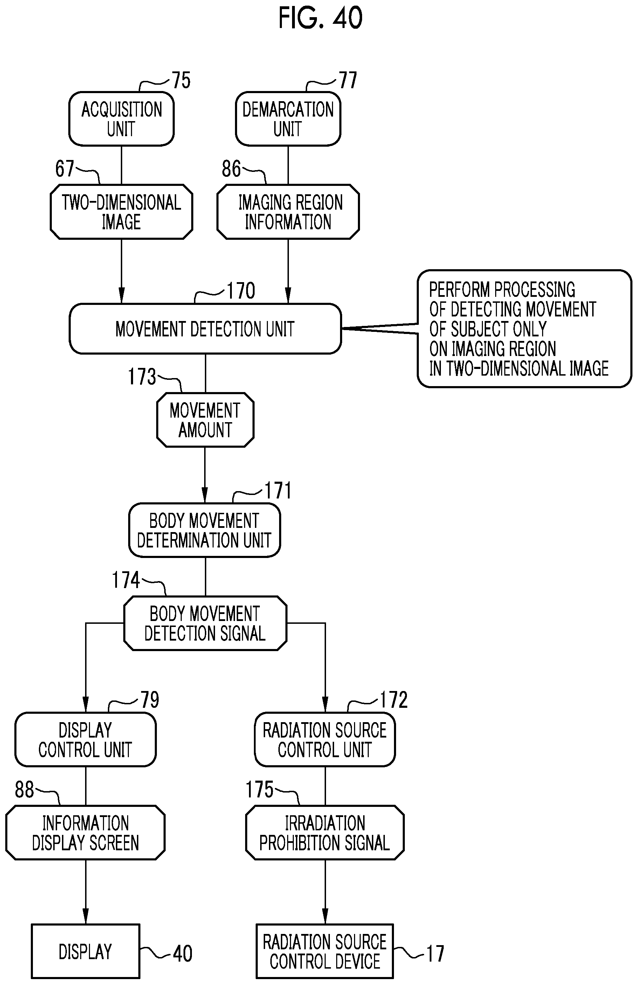

As an example, as shown in Fig. 40, the CPU 57 of the console 14 according to Embodiment 3_1 functions as a movement detection unit 170, a body movement determination unit 171, and a radiation source control unit 172 in addition to the processing units 75 to 79 (the extraction unit 76 and the determination unit 78 are not shown) according to Embodiment 1.

The movement detection unit 170 receives the two-dimensional image 67 input from the acquisition unit 75, and the imaging region information 86 input from the demarcation unit 77. The movement detection unit 170 performs processing of detecting the movement of the subject H only on the imaging region IR of the two-dimensional image 67. The movement detection unit 170 outputs the amount of the detected movement of the subject H (hereinafter, referred to as a movement amount) 173 to the body movement determination unit 171.

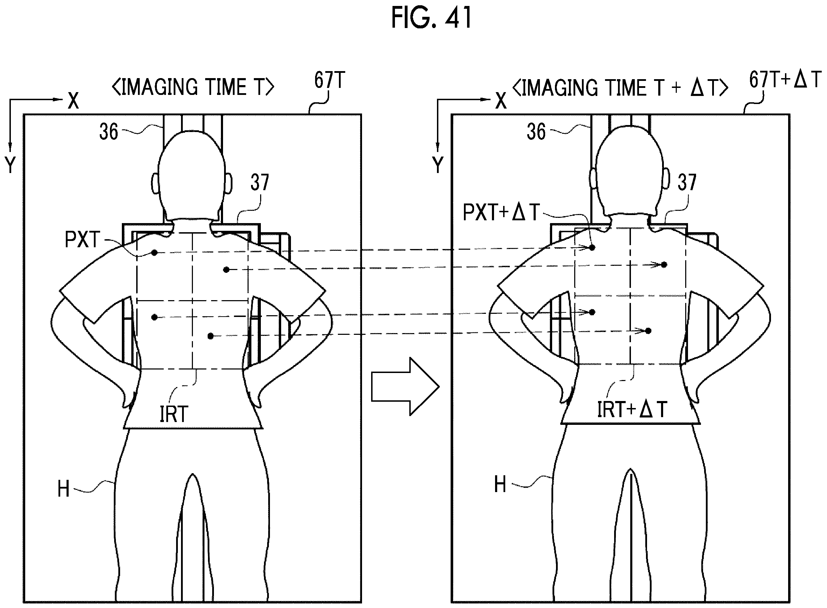

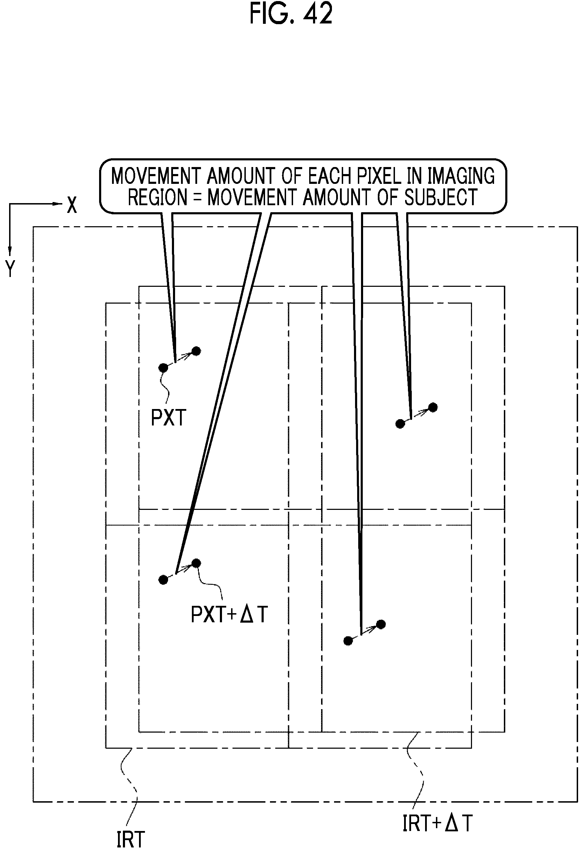

As shown in Figs. 41 and 42 as an example, the movement detection unit 170 detects the movement amount 173 from an imaging region IRT of a two-dimensional image 67T at an imaging time point T and an imaging region IRT+ΔT of the two-dimensional image 67T+ΔT at an imaging time point T+ΔT (ΔT is, for example, a frame interval of the two-dimensional image 67). More specifically, the movement detection unit 170 calculates, as the movement amount 173, a representative value of the movement amount of each pixel PXT+ΔT in the imaging region IRT+ΔT with respect to each pixel PXT in the imaging region IRT. The representative value is, for example, an average value or a mode value. This processing is based on the assumption that the brightness of the object shown in the image is not changed between the images that are temporally continuous, and the movement amount 173 of the adjacent pixels has substantially the same value. The two-dimensional image 67T and the two-dimensional image 67T+ΔT are examples of "two pieces of the two-dimensional images captured at different imaging time points" according to the technology of the present disclosure.

Returning to Fig. 40, the body movement determination unit 171 compares the movement amount 173 with a preset threshold value. In a case in which the movement amount is equal to or greater than the threshold value, the body movement determination unit 171 determines that the body movement that is not allowable in the radiography has occurred in the subject H, and outputs a body movement detection signal 174 indicating the determination result to the display control unit 79 and the radiation source control unit 172. On the other hand, in a case in which the movement amount is less than the threshold value, the body movement determination unit 171 does not perform any processing.

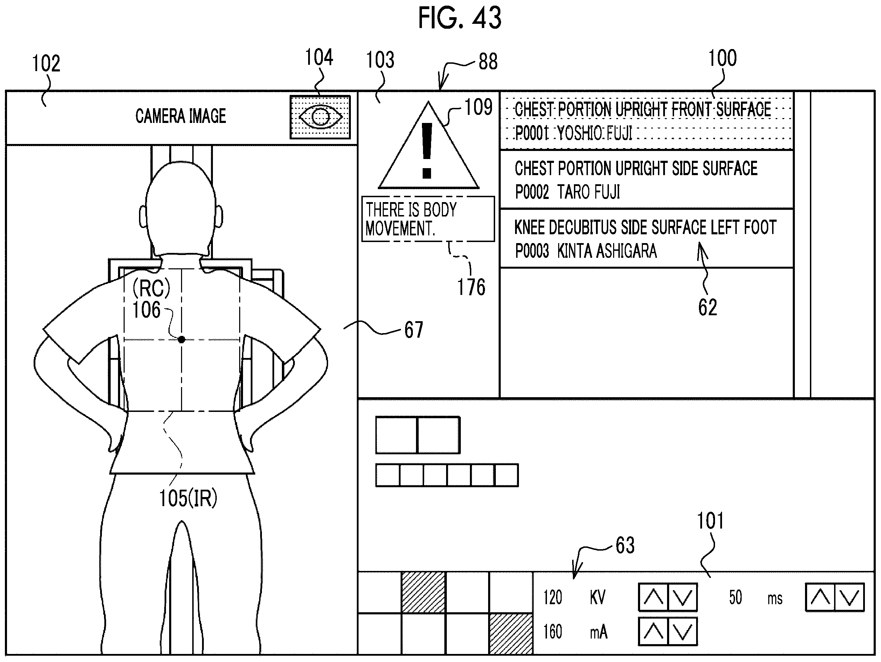

In a case in which the body movement detection signal 174 is input from the body movement determination unit 171, the display control unit 79 performs control of displaying the information display screen 88 shown in Fig. 43 as an example on the display 40. In Fig. 43, the exclamation mark 109 and a message 176 indicating that the body movement that is not allowable in the radiography has occurred in the subject H are displayed in the display region 103 of the information display screen 88. The operator OP instructs the subject H not to move the body by making an announcement to the speaker 27 of the radiography room 25 via the microphone 28.

Returning to Fig. 40 again, in a case in which the body movement detection signal 174 is input from the body movement determination unit 171, the radiation source control unit 172 transmits an irradiation prohibition signal 175 to the radiation source control device 17. In a case in which the irradiation prohibition signal 175 is received, the radiation source control device 17 prevents the radiation source 16 from emitting the radiation R even in a case in which an instruction to start the irradiation with the radiation R is given through the irradiation switch 19.

As described above, in Embodiment 3_1, the movement detection unit 170 performs the processing of detecting the movement of the subject H only on the imaging region IR. Therefore, the movement of the body part other than the imaging part, in this case, the movement of the head portion, the arm, the hand, the waist, the foot, or the like other than the chest portion, which is not related to the radiography of the body part other than the imaging part, is not captured, and only the body movement of the imaging part can be purely detected. In addition, the processing speed can be increased as compared with a case in which the processing is performed on the entire two-dimensional image 67.

In a case in which the movement amount 173 is equal to or greater than the preset threshold value, the display control unit 79 outputs the fact that the movement amount 173 is equal to or greater than the threshold value by displaying the message 176 in the display region 103 of the information display screen 88 as shown in Fig. 43. Therefore, it is possible to notify the operator OP that the body movement that is not allowable in the radiography has occurred in the subject H.

In addition, in a case in which the movement amount 173 is equal to or greater than the preset threshold value, the radiation source control unit 172 performs control of preventing the radiation source 16 from emitting the radiation R by transmitting the irradiation prohibition signal 175 to the radiation source control device 17. Therefore, it is possible to prevent the subject H from being exposed to unnecessary radiation due to the imaging failure caused by the radiography being performed while the body movement that is not allowable in the radiography has occurred in the subject H. In addition, the probability of obtaining the radiation image 66 suitable for the diagnosis is increased.

The processing of detecting the movement of the subject H is processing of calculating the movement amount of the object shown in two pieces of the two-dimensional images 67 captured at different imaging time points. Therefore, relatively small movements such as breathing can be accurately detected in addition to relatively large movements such as shaking of the body.

### [Embodiment 3_2]

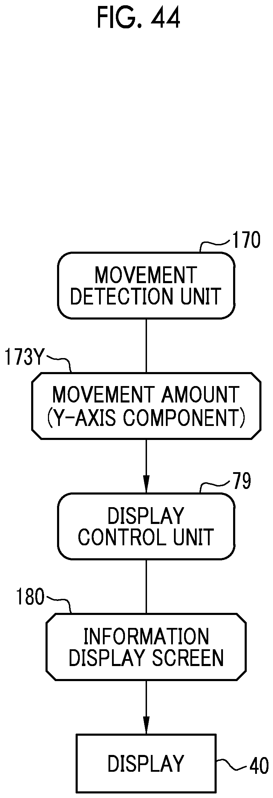

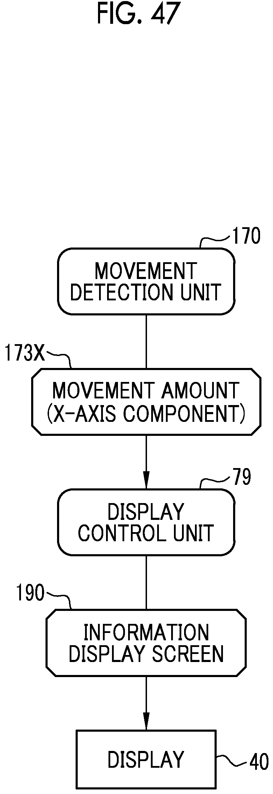

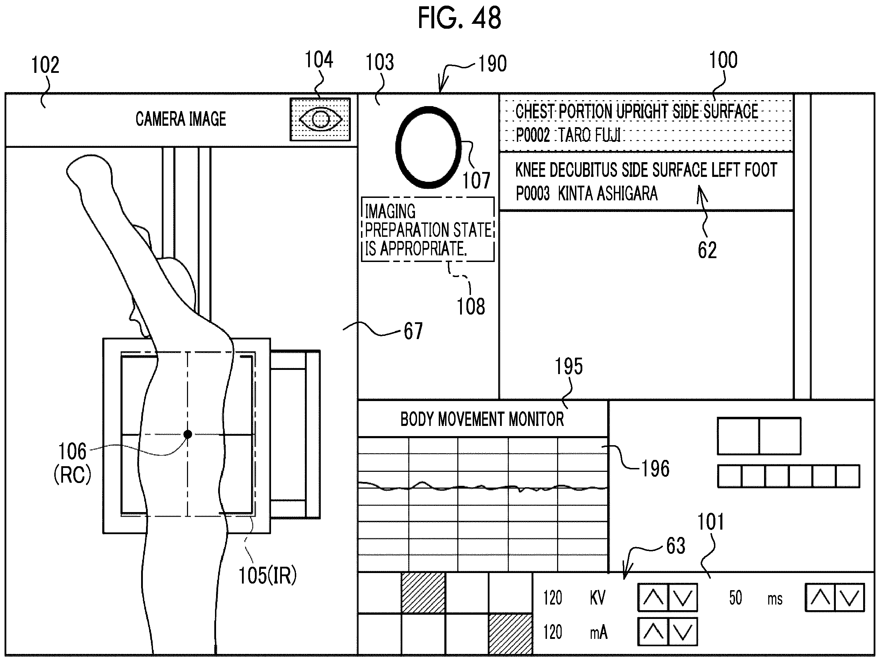

In Embodiment 3_2, control of displaying a temporal change in a component on a specific axis of the movement amount 173 on the display 40 is performed. Figs. 44 to 46 show a case of the chest portion upright front surface imaging, and Figs. 47 and 48 show a case of the chest portion upright side surface imaging.

In a case of the chest portion upright front surface imaging, as shown in Fig. 44 as an example, the movement detection unit 170 outputs a Y-axis component 173Y of the movement amount to the display control unit 79. The Y-axis component 173Y of the movement amount is a representative value of the Y-axis component of the movement amount of each pixel PXT in the imaging region IRT+ΔT with respect to each pixel PXT in the imaging region IRT as shown in Fig. 41 and Fig. 42. The display control unit 79 performs control of displaying an information display screen 180 showing a temporal change in the Y-axis component 173Y of the movement amount on the display 40. The Y-axis is an example of a "specific axis" according to the technology of the present disclosure.

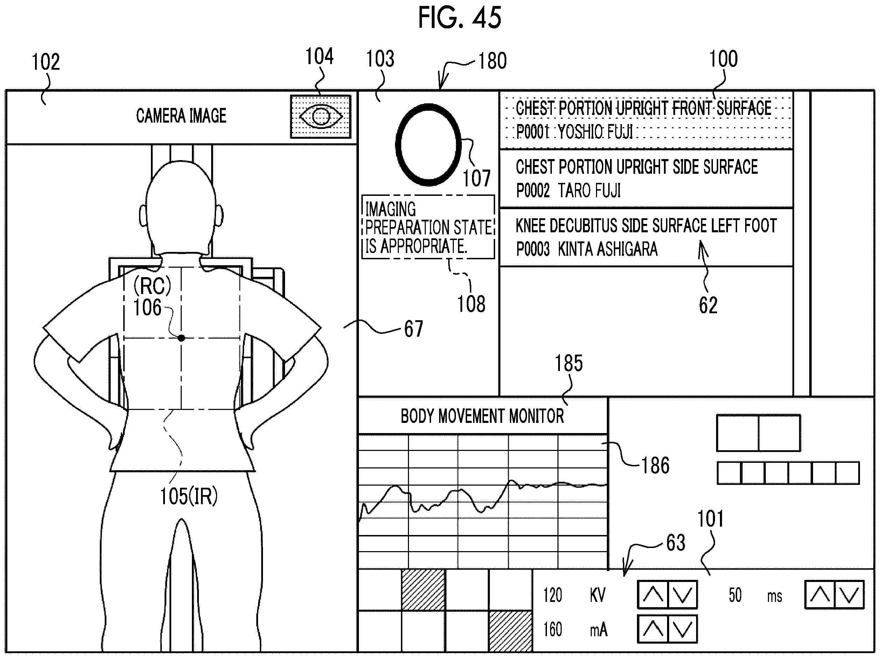

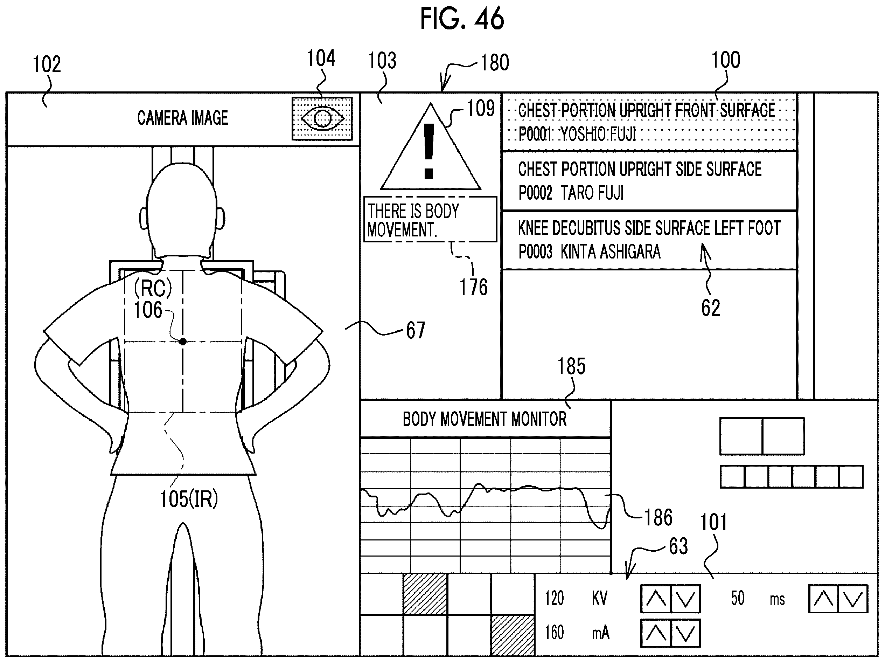

As shown in Figs. 45 and 46 as an example, a display region 185 is provided on the information display screen 180, in addition to the display regions 100 to 103. A movement amount graph 186 is displayed in the display region 185. The movement amount graph 186 is a graph in which a vertical axis indicates the Y-axis component 173Y of the movement amount and a horizontal axis indicates the time. That is, the movement amount graph 186 shows the temporal change in the Y-axis component 173Y of the movement amount.

Fig. 45 shows a moment immediately after the subject H inhales and holds the breath in response to the instruction of the operator OP. In this case, the Y-axis component 173Y of the movement amount is substantially constant. On the other hand, Fig. 46 shows a case in which the subject H exhales the breath by mistake and the body movement determination unit 171 determines that the body movement that is not allowable in the radiography has occurred in the subject H.

In a case of the chest portion upright side surface imaging, as shown in Fig. 47 as an example, the movement detection unit 170 outputs an X-axis component 173X of the movement amount to the display control unit 79. The X-axis component 173X of the movement amount is a representative value of the X-axis component of the movement amount of each pixel PXT in the imaging region IRT+ΔT with respect to each pixel PXT in the imaging region IRT as shown in Fig. 41 and Fig. 42. The display control unit 79 performs control of displaying an information display screen 190 showing a temporal change in the X-axis component 173X of the movement amount on the display 40. In this case, the X-axis is an example of a "specific axis" according to the technology of the present disclosure.

As shown in Fig. 48 as an example, a movement amount graph 196 displayed in a display region 195 of the information display screen 190 is a graph in which a vertical axis indicates the X-axis component 173X of the movement amount and a horizontal axis indicates a time. That is, the movement amount graph 196 in this case shows the temporal change in the X-axis component 173X of the movement amount. Fig. 48 shows a moment immediately after the subject H inhales and holds the breath in response to the instruction of the operator OP. In this case, the X-axis component 173X of the movement amount is substantially constant.

As described above, in Embodiment 3_2, the display control unit 79 performs control of displaying the temporal change in the component on the specific axis of the movement amount 173 of the subject H on the display 40. Therefore, the operator OP can know the temporal change in the component on the specific axis of the movement amount 173 of the subject H. It is possible to prevent the subject H from being unnecessarily exposed to exposure due to an imaging failure due to the radiography performed with insufficient breath holding of the subject H. In addition, the probability of obtaining the radiation image 66 suitable for the diagnosis is increased.

It should be noted that the reason for displaying the temporal change in the Y-axis component 173Y of the movement amount in a case of the chest portion upright front surface imaging and displaying the temporal change in the X-axis component 173X of the movement amount in a case of the chest portion upright side surface imaging is as follows. First, in a case of the chest portion upright front surface imaging, it is considered that the body movement due to the respiration is caused mainly in the upper and lower directions of the diaphragm along the Y-axis direction. On the other hand, in a case of the chest portion upright side surface imaging, it is considered that the body movement due to the respiration is caused mainly by the expansion and contraction of the abdomen portion along the X-axis direction.

Instead of or in addition to outputting the information corresponding to the determination result 87 via the information display screen 88, the information corresponding to the determination result 87 may be output by voice. The information corresponding to the determination result 87 may be output by an indicator such as a warning lamp.

The feature points 91 that are not related to the extraction of the portion of interest POI, for example, the right and left external auditory canal points, the right and left elbow joint points, and the right and left wrist joint points according to Embodiment 1 described above, do not have to be extracted. The feature point 91 is extracted and then the portion of interest POI is extracted, but the present disclosure is not limited to this. The portion of interest POI may be directly extracted from the two-dimensional image 67 or the like without extracting the feature point 91, by using a machine learning model or the like.

Examples of the portion of interest POI include the following in addition to the prominent vertebra point and the like described as an example. That is, examples thereof include laryngeal prominence (thyroid cartilage) in a case in which the imaging part is the head portion, suprasternal space, shoulder blade lower end, xiphoid process, and rib lower edge in which the imaging part is the chest portion, iliac crest upper edge (line connecting right and left iliac crests (Jacoby's line)), anterior superior iliac spine, pubic symphysis/greater trochanter, and coccyx in a case in which the imaging part is the waist portion. These examples are so-called body surface indicators.

The electronic cassette 13 has been described as an example of the radiation image detector, but the present disclosure is not limited to this. A radiation image detector provided in the upright imaging tables 12 and 145 or the decubitus imaging table 120 may be used. In addition, the imaging table is not limited to the upright imaging tables 12 and 145 or the decubitus imaging table 120 as examples. A seat imaging table for performing the radiography on the subject H in a seated posture may be used. Further, the radiation source 16 may be a radiation source of a type that is suspended from a ceiling of the radiography room 25.

The display may be attached to the upright imaging tables 12 and 145, the radiation source table 15, or the decubitus imaging table 120, to display various screens, such as the information display screens 88, 180, and 190, on the display. In this way, it is possible to confirm the information display screen 88 and the like even in the radiography room 25. In addition, a guide for the positioning or the like can be displayed on the subject H.

Various screens, such as the information display screens 88, 180, and 190, may be transmitted to a portable terminal, such as a tablet terminal owned by the operator OP, from the console 14, for example, in a form of screen data for web distribution created by markup language, such as extensible markup language (XML). In this case, the portable terminal reproduces various screens to be displayed on the web browser based on the screen data and displays the screens on the display. It should be noted that another data description language, such as Javascript (registered trademark) object notation (JSON), may be used instead of the XML.

It is possible to make various modifications with respect to the hardware configuration of the computer constituting the imaging support apparatus according to the technology of the present disclosure. For example, the imaging support apparatus can be composed of a plurality of computers separated as hardware in order to improve the processing capacity and the reliability. For example, the functions of the acquisition unit 75, the extraction unit 76, and the demarcation unit 77, and the functions of the determination unit 78 and the display control unit 79 are distributed to two computers. In this case, the two computers constitute the imaging support apparatus.

As described above, the hardware configuration of the computer of the imaging support apparatus can be appropriately changed in accordance with required performance, such as processing capacity, safety, and reliability. Further, it is needless to say that, in addition to the hardware, an application program, such as the operation program 70, can be duplicated or distributed and stored in a plurality of storages for the purpose of securing the safety and the reliability.

In each embodiment described above, for example, as the hardware structure of the processing unit that executes various types of processing, such as the acquisition unit 75, the extraction unit 76, the demarcation unit 77, the determination unit 78, the display control unit 79, the position adjustment unit 160, the angle adjustment unit 165, the movement detection unit 170, the body movement determination unit 171, and the radiation source control unit 172, various processors shown below can be used. As described above, the various processors include, in addition to the CPU 57, which is a general-purpose processor that executes software (operation program 70) to function as the various processing units, a programmable logic device (PLD), which is a processor of which a circuit configuration can be changed after the manufacturing, such as a field programmable gate array (FPGA), a dedicated electric circuit, which is a processor having a circuit configuration designed exclusively for executing specific processing, such as an application specific integrated circuit (ASIC), and the like.

One processing unit may be composed of one of various processors described above or may be composed of a combination of two or more processors (for example, a combination of a plurality of ASICs and/or a combination of an ASIC and a FPGA) of the same type or different types. In addition, a plurality of the processing units may be composed of one processor.

As an example in which the plurality of processing units are composed of one processor, firstly, as represented by a computer, such as a client and a server, there is a form in which one processor is composed of a combination of one or more CPUs and software, and the processor functions as the plurality of processing units. Second, as represented by a system-on-chip (SoC) or the like, there is a form in which a processor, which realizes the functions of the entire system including the plurality of processing units with a single integrated circuit (IC) chip, is used. As described above, various processing units are composed of one or more of the various processors as the hardware structure.

Further, as the hardware structure of these various processors, more specifically, it is possible to use an electric circuit (circuitry) in which circuit elements, such as semiconductor elements, are combined.

The technology of the present disclosure can also be appropriately combined with various embodiments and/or various modification examples described above. In addition, it is needless to say that the present disclosure is not limited to each of the embodiments described above, various configurations can be adopted as long as the configuration does not deviate from the gist. Further, the technology of the present disclosure includes, in addition to the program, a storage medium that stores the program in a non-transitory manner.

The described contents and shown contents above are the detailed description of the parts according to the technology of the present disclosure, and are merely an example of the technology of the present disclosure. For example, the above description of the configuration, the function, the action, and the effect are the description of examples of the configuration, the function, the action, and the effect of the parts according to the technology of the present disclosure. Accordingly, it is needless to say that unnecessary parts may be deleted, new elements may be added, or replacements may be made with respect to the described contents and shown contents above within a range that does not deviate from the gist of the technology of the present disclosure. In addition, in order to avoid complications and facilitate grasping the parts according to the technology of the present disclosure, in the described contents and shown contents above, the description of technical general knowledge and the like that do not particularly require description for enabling the implementation of the technology of the present disclosure are omitted.

In the present specification, "A and/or B" is synonymous with "at least one of A or B". That is, "A and/or B" means that it may be only A, only B, or a combination of A and B. In addition, in the present specification, also in a case in which three or more matters are associated and expressed by "and/or", the same concept as "A and/or B" is applied.

All documents, patent applications, and technical standards described in the present specification are incorporated herein by reference to the same extent as in a case in which each document, each patent application, and each technical standard are specifically and individually described by being incorporated by reference.

## Claims

1. An imaging support apparatus comprising:
a processor,
wherein the processor
acquires a two-dimensional image obtained by imaging a subject who undergoes radiography via a camera,
extracts a portion of interest of the subject included in the two-dimensional image,
demarcates an imaging region to be imaged by the radiography in the two-dimensional image based on the portion of interest,
determines whether or not an imaging preparation state before the radiography is appropriate based on the imaging region, and
outputs information in accordance with a determination result.

2. The imaging support apparatus according to claim 1,
wherein the processor determines that the imaging preparation state is not appropriate in a case in which an unnecessary part for the radiography is included in the imaging region or a preset peripheral region of the imaging region.

3. The imaging support apparatus according to claim 1 or 2,
wherein the processor determines that the imaging preparation state is not appropriate in a case in which a deviation between a center of the imaging region and an irradiation center of radiation is equal to or greater than a preset threshold value.

4. The imaging support apparatus according to any one of claims 1 to 3,
wherein the processor performs control of displaying, on a display, a deviation amount between a center of the imaging region and an irradiation center of radiation in a real space.

5. The imaging support apparatus according to any one of claims 1 to 4,
wherein the processor performs control of displaying, on a display, the two-dimensional image on which a frame indicating the imaging region is superimposed.

6. The imaging support apparatus according to any one of claims 1 to 5,
wherein the processor
detects an angle of an imaging part to be imaged by the radiography based on the portion of interest, and
determines that the imaging preparation state is not appropriate in a case in which the angle is out of a preset range.

7. The imaging support apparatus according to any one of claims 1 to 6,
wherein the processor
detects an angle of an imaging part to be imaged by the radiography based on the portion of interest, and
performs control of adjusting an angle of a radiation source that emits radiation with respect to the subject according to the angle.

8. The imaging support apparatus according to any one of claims 1 to 7,
wherein the processor
detects a degree of inclination of a posture of the subject based on the portion of interest, and
determines that the imaging preparation state is not appropriate in a case in which the degree of inclination is out of a preset range.

9. The imaging support apparatus according to any one of claims 1 to 8,
wherein the processor performs control of adjusting positions of a radiation source that emits radiation and a radiation image detector that receives the radiation to detect a radiation image, to reduce a deviation between a center of the imaging region and an irradiation center of the radiation.

10. The imaging support apparatus according to any one of claims 1 to 9,
wherein the processor performs control of displaying, on a display, adjustment amounts of positions of a radiation source that emits radiation and a radiation image detector that receives the radiation to detect a radiation image, the adjustment amounts being for reducing a deviation between a center of the imaging region and an irradiation center of the radiation.

11. The imaging support apparatus according to any one of claims 1 to 10,
wherein the processor performs processing of detecting movement of the subject only on the imaging region.

12. The imaging support apparatus according to claim 11,
wherein the processor outputs, in a case in which a movement amount of the subject is equal to or greater than a preset threshold value, a fact that the movement amount of the subject is equal to or greater than the threshold value.

13. The imaging support apparatus according to claim 11 or 12,
wherein the processor performs, in a case in which a movement amount of the subject is equal to or greater than a preset threshold value, control of preventing a radiation source from emitting radiation.

14. The imaging support apparatus according to any one of claims 11 to 13,
wherein the processor performs control of displaying, on a display, a temporal change in a component on a specific axis of a movement amount of the subject.

15. The imaging support apparatus according to any one of claims 11 to 14,
wherein the processing of detecting the movement of the subject is processing of calculating a movement amount of an object shown in two pieces of the two-dimensional images captured at different imaging time points.

16. An operation method of an imaging support apparatus, the method comprising:
acquiring a two-dimensional image obtained by imaging a subject who undergoes radiography via a camera;
extracting a portion of interest of the subject included in the two-dimensional image;
demarcating an imaging region to be imaged by the radiography in the two-dimensional image based on the portion of interest;
determining whether or not an imaging preparation state before the radiography is appropriate based on the imaging region; and
outputting information in accordance with a determination result.

17. An operation program of an imaging support apparatus, the program causing a computer to execute a process comprising:
acquiring a two-dimensional image obtained by imaging a subject who undergoes radiography via a camera;
extracting a portion of interest of the subject included in the two-dimensional image;
demarcating an imaging region to be imaged by the radiography in the two-dimensional image based on the portion of interest;
determining whether or not an imaging preparation state before the radiography is appropriate based on the imaging region; and
outputting information in accordance with a determination result.
